(19) Europäisches Patentamt
European Patent Office
Office européen des brevets

(11) **EP 3 063 112 B1**

(12) **EUROPÄISCHE PATENTSCHRIFT**

(45) Veröffentlichungstag und Bekanntmachung des Hinweises auf die Patenterteilung:
**13.12.2017 Patentblatt 2017/50**

(21) Anmeldenummer: **14790068.2**

(22) Anmeldetag: **27.10.2014**

(51) Int Cl.:
*C07C 5/327* *(2006.01)*    *C07C 5/333* *(2006.01)*
*C07C 11/167* *(2006.01)*

(86) Internationale Anmeldenummer:
**PCT/EP2014/072978**

(87) Internationale Veröffentlichungsnummer:
**WO 2015/063019 (07.05.2015 Gazette 2015/18)**

(54) **VERFAHREN ZUR HERSTELLUNG VON 1,3-BUTADIEN AUS N-BUTENEN DURCH OXIDATIVE DEHYDRIERUNG**

METHOD FOR PREPARING 1,3-BUTADIENE FROM N-BUTENES BY OXIDATIVE DEHYDRATION

PROCÉDÉ DE FABRICATION DE 1,3-BUTADIÈNE À PARTIR DE N-BUTÈNE PAR DÉSHYDRATATION OXYDATIVE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.10.2013 EP 13190897**

(43) Veröffentlichungstag der Anmeldung:
**07.09.2016 Patentblatt 2016/36**

(73) Patentinhaber: **BASF SE**
**67056 Ludwigshafen am Rhein (DE)**

(72) Erfinder:
• **GRÜNE, Philipp**
  **68161 Mannheim (DE)**
• **HAMMEN, Oliver**
  **67587 Wintersheim (DE)**
• **SCHMITT, Christine**
  **68163 Mannheim (DE)**
• **BALEGEDDE RAMACHANDRAN, Ragavendra Prasad**
  **67069 Ludwigshafen (DE)**

• **JOSCH, Jan Pablo**
  **67434 Neustadt (DE)**
• **WALSDORFF, Christian**
  **67061 Ludwigshafen (DE)**

(56) Entgegenhaltungen:
**US-A1- 2012 130 137**

• **DATABASE WPI Week 201230 Thomson Scientific, London, GB; AN 2012-E48215 XP002724650, & JP 2012 072086 A (ASAHI KASEI KK) 12. April 2012 (2012-04-12) in der Anmeldung erwähnt**

• **DATABASE WPI Week 201107 Thomson Scientific, London, GB; AN 2011-A58751 XP002724651, & JP 2011 006381 A (MITSUBISHI CHEM CORP) 13. Januar 2011 (2011-01-13) in der Anmeldung erwähnt**

• **DATABASE WPI Week 198531 Thomson Scientific, London, GB; AN 1985-187149 XP002724652, & JP S60 115532 A (NIPPON ZEON KK) 22. Juni 1985 (1985-06-22)**

## Beschreibung

Die Erfindung betrifft ein Verfahren zur Herstellung von 1,3-Butadien aus n-Butenen durch oxidative Dehydrierung (ODH).

Butadien (1,3-Butadien) ist eine bedeutende Grundchemikalie und wird beispielsweise zur Herstellung von Synthesekautschuken (Butadien-Homopolymere, Styrol-Butadien-Kautschuk oder Nitril-Kautschuk) oder zur Herstellung von thermoplastischen Terpolymeren (AcrylnitrilButadien-Styrol-Copolymere) eingesetzt. Butadien wird ferner zu Sulfolan, Chloropren und 1,4-Hexamethylendiamin (über 1,4-Dichlorbuten und Adipinsäuredinitril) umgesetzt. Durch Dimerisierung von Butadien kann ferner Vinylcyclohexen erzeugt werden, welches zu Styrol dehydriert werden kann.

Butadien kann durch thermische Spaltung (Steam-Cracken) gesättigter Kohlenwasserstoffe hergestellt werden, wobei üblicherweise von Naphtha als Rohstoff ausgegangen wird. Beim Steam-Cracken von Naphtha fällt ein Kohlenwasserstoff-Gemisch aus Methan, Ethan, Ethen, Acetylen, Propan, Propen, Propin, Allen, Butanen, Butenen, Butadien, Butinen, Methylallen, $C_5$- und höheren Kohlenwasserstoffen an.

Butadien kann auch durch oxidative Dehydrierung von n-Butenen (1-Buten und/oder 2-Buten) in Anwesenheit von molekularem Sauerstoff erhalten werden. Als Einsatzgasstrom für die oxidative Dehydrierung (Oxidehydrierung, ODH) von n-Butenen zu Butadien kann jedes beliebige n-Butene enthaltende Gemisch benutzt werden. Beispielsweise kann eine Fraktion verwendet werden, die als Hauptbestandteil n-Butene (1-Buten und/oder 2-Buten) enthält und aus der $C_4$-Fraktion eines Naphtha-Crackers durch Abtrennen von Butadien und Isobuten erhalten wurde. Des Weiteren können auch Gasgemische als Einsatzgasstrom eingesetzt werden, die 1-Buten, cis-2-Buten, trans-2-Buten oder deren Gemische umfassen und durch Dimerisierung von Ethylen erhalten wurden. Ferner können als Einsatzgasstrom n-Butene enthaltende Gasgemische eingesetzt werden, die durch katalytisches Wirbelschichtcracken (Fluid Catalytic Cracking, FCC) erhalten wurden.

Neben n-Butenen und molekularem Sauerstoff enthält das Reaktionsgasgemisch in der Regel inerte Komponenten. Inerte Komponenten bedeutet hier, dass sie unter den Reaktionsbedingungen der ODH zu weniger als 90 % umgesetzt werden. Inerte Komponenten sind beispielsweise Wasserdampf und Stickstoff, aber beispielsweise auch Alkane wie Methan. Das Molmengenverhältnis der inerten Komponente zu molekularem Sauerstoff ist hierbei in der Regel höher als es in Luft vorliegt, vor allem um der Gefahr von Explosionen vorzubeugen. Dies kann beispielsweise geschehen, indem Luft als sauerstoffhaltiges Gas eingesetzt wird und mit molekularem Stickstoff verdünnt wird. Allerdings ist die Bereitstellung von großen Mengen an aufkonzentriertem Stickstoff teuer und unter wirtschaftlichen Gesichtspunkten unvorteilhaft. Weiterhin kann dies geschehen, indem eine an molekularem Sauerstoff abgereicherte Luft (Magerluft) als sauerstoffhaltiges Gas eingesetzt wird. Weiterhin kann dies geschehen, indem Luft mit Magerluft verdünnt wird.

Verfahren zur oxidativen Dehydrierung von Butenen zu Butadien sind grundsätzlich bekannt.

US 2012/0130137A1 beispielsweise beschreibt ein solches Verfahren unter Verwendung von Katalysatoren die Oxide von Molybdän, Bismuth und in der Regel weiteren Metallen enthalten. Für die nachhaltige Aktivität solcher Katalysatoren für die oxidative Dehydrierung ist ein kritisches Mindestmaß an Sauerstoffpartialdruck in der Gasatmosphäre erforderlich, um eine zu weitgehende Reduktion und damit einen Performanceverlust der Katalysatoren zu vermeiden. Aus diesem Grund kann in der Regel auch nicht mit einem stöchiometrischen Sauerstoffeinsatz oder vollständigem Sauerstoff-Umsatz im Oxidehydrierungsreaktor (ODH-Reaktor) gearbeitet werden. In der US 2012/0130137 wird zum Beispiel ein Sauerstoffgehalt von 2,5 bis 8 Vol.-% im Ausgangsgas beschrieben.

Das $N_2/O_2$ Verhältnis im Reaktionsgasgemisch wird auf den gewünschten Wert eingestellt, in dem Luft als sauerstoffhaltiges Gas mit Stickstoffgas verdünnt wird.

Die Notwendigkeit eines Sauerstoffüberschusses für solche Katalysatorsysteme ist allgemein bekannt und schlägt sich in den Verfahrensbedingungen bei Verwendung derartiger Katalysatoren nieder. Stellvertretend seien die neueren Arbeiten von Jung et al. (Catal. Surv. Asia 2009, 13, 78-93; DOI 10.1007/s10563-009-9069-5 und Applied Catalysis A: General 2007, 317, 244-249; DOI 10.1016/j.apcata.2006.10.021) genannt.

Das Vorliegen von Sauerstoff neben Butadien nach der ODH-Reaktorstufe im Aufarbeitungsteil derartiger, unter Sauerstoff-Überschuss betriebener Verfahren ist jedoch mit Risiken behaftet. Insbesondere in flüssiger Phase ist die Bildung und Anreicherung organischer Peroxide zu überprüfen. Diese Risiken wurden zum Beispiel von D. S. Alexander (Industrial and Engineering Chemistry 1959, 51, 733 - 738) diskutiert.

In JP 2011-006381 A der Firma Mitsubishi wird das Risiko der Peroxidbildung im Aufarbeitungsteil eines Verfahrens zur Herstellung von konjugierten Alkadienen angesprochen. Zur Lösung wird der Zusatz von Polymerisationsinhibitoren zu den Absorptionslösungen für die Prozessgase und die Einstellung eines maximalen Peroxidgehalts von 100 Gewichts-ppm durch Erhitzen der Absorptionslösungen beschrieben. Allerdings werden keine Angaben zur Vermeidung oder Kontrolle von Peroxiden in vorgelagerten Verfahrensschritten gemacht. Kritisch ist insbesondere der Kühlschritt des ODH-Reaktoraustrags mit einem Wasser-Quench zu sehen. Gebildete organische Peroxide sind kaum in Wasser löslich, so dass sie sich abscheiden und in fester oder flüssiger Form im Apparat anreichern können, statt mit dem wässrigen Purge-Strom des Quenches ausgetragen zu werden. Gleichzeitig ist die Temperatur des Wasser-Quench nicht so hoch, als dass von einem ausreichend hohen und stetigen Abbau der gebildeten Peroxide ausgegangen werden kann.

Bei der katalytischen oxidativen Dehydrierung können hochsiedende Nebenkomponenten wie beispielsweise Maleinsäureanhydrid, Phthalsäureanhydrid, Benzaldehyd, Benzoesäure, Ethylbenzol, Styrol, Fluorenon, Anthrachinon und andere gebildet werden. Solche Ablagerungen können zu Verstopfungen und zu einem Druckverlustanstieg im Reaktor oder hinter dem Reaktor im Bereich der Aufarbeitung führen und so einen geregelten Betrieb stören. Ablagerungen der genannten hochsiedenden Nebenkomponenten können auch die Funktion von Wärmetauschern beeinträchtigen oder bewegte Apparate wie Kompressoren schädigen. Wasserdampfflüchtige Verbindungen wie Fluorenon können durch einen mit Wasser betriebenen Quench-Apparat durchschlagen und sich dahinter in den Gasaustragsleitungen niederschlagen. Damit besteht grundsätzlich auch die Gefahr, dass feste Ablagerungen in nachgeschaltete Apparateteile, wie beispielsweise Kompressoren, gelangen und dort Schaden anrichten.

In US 2012/0130137A1 Absatz [0122] wird auch auf die Problematik hochsiedender Nebenprodukte hingewiesen. Insbesondere werden Phthalsäureanhydrid, Anthrachinon und Fluorenon genannt, die typischerweise in Konzentrationen von 0,001 bis 0,10 Vol.-% im Produktgas vorlägen. In der US 2012/0130137A1 Absatz [0124] - [0126] wird empfohlen, die heißen Reaktoraustragsgase direkt durch Kontakt mit einer Kühlflüssigkeit (Quench-Turm) auf üblicherweise zunächst 5 bis 100 °C abzukühlen. Als Kühlflüssigkeiten werden Wasser oder wässrige AlkaliLösungen genannt. Ausdrücklich wird die Problematik von Verstopfungen im Quench durch Hochsieder aus dem Produktgas oder durch Polymerisationsprodukte hochsiedender Nebenprodukte aus dem Produktgas erwähnt, weshalb es vorteilhaft sei, dass hochsiedende Nebenprodukte so wenig wie möglich aus dem Reaktionsteil in den Kühlungsteil (Quench) mitgetragen werden.

In KR 2013-0036467 und KR 2013-0036468 wird ebenfalls empfohlen, die heißen Reaktoraustragsgase direkt durch Kontakt mit einem Kühlmittel abzukühlen. Als Kühlmittel werden in Wasser lösliche organische Kühlmittel verwendet, um die Nebenkomponenten besser abzukühlen.

In JP 2011-001341A wird für ein Verfahren zur oxidativen Dehydrierung von Alkenen zu konjugierten Alkadienen eine zweistufige Kühlung beschrieben. Dabei wird das Produktaustragsgas der oxidativen Dehydrierung zunächst auf eine Temperatur zwischen 300 und 221 °C eingestellt und dann auf eine Temperatur zwischen 99 und 21 °C weiter abgekühlt. In den Absätzen [0066] ff. wird beschrieben, dass zur Einstellung der Temperatur zwischen 300 und 221 °C bevorzugt Wärmetauscher eingesetzt werden, wobei aber auch ein Teil der Hochsieder aus dem Produktgas in diesen Wärmetauschern ausfallen könnten. In der JP 2011-001341A wird deshalb ein gelegentliches Auswaschen von Ablagerungen aus den Wärmetauschern mit organischen oder wässrigen Lösungsmitteln beschrieben. Als Lösungsmittel werden beispielsweise aromatische Kohlenwasserstoffe wie Toluol oder Xylol oder ein alkalisches wässriges Lösungsmittel wie beispielsweise die wässrige Lösung von Natriumhydroxid beschrieben. Um ein zu häufiges Abstellen des Verfahrens zur Reinigung des Wärmetauschers zu vermeiden, wird in der der JP2011-001341A ein Aufbau mit zwei parallel angeordneten Wärmetauschern beschrieben, die jeweils abwechselnd betrieben oder gespült werden (sogenannte A/B-Fahrweise).

In JP 2010-90083 A wird ein Verfahren zur oxidativen Dehydrierung von n-Butenen zu Butadien beschrieben, bei dem das Produktgas der oxidativen Dehydrierung abgekühlt und entwässert wird. Aus dem C4-haltigen Einsatzgasstrom werden anschließend Butadien sowie nicht umgesetzte Butene und Butan in einem Lösungsmittel absorbiert. Das nicht vom Lösungsmittel absorbierte Restgas wird anschließen durch Verbrennung entsorgt. Wird ein Lösungsmittel wie Toluol mit einem niedrigen Siedepunkt als Absorptionsmittel verwendet, so wird dieses zwecks Vermeidung von Lösungsmittelverlusten aus dem Restgasstrom durch Absorption in einem Lösungsmittel mit hohem Siedepunkt, beispielsweise Decan, wiedergewonnen.

In JP 2012072086 wird in Absatz [0014] beschrieben, dass als sauerstoffhaltiges Gas ein Gas, bei dem die Kohlenwasserstoffe, wie Butadien, n-Buten, n-Butan, iso-Butan von dem Produktgasgemisch abgetrennt worden sind, in die Oxidehydrierung zurückgeführt werden kann. Die Schrift macht keine Aussagen dazu, wie ein solcher Rückführgasstrom gewonnen wird, und welche Verunreinigungen darin enthalten sind.

Aufgabe der vorliegenden Erfindung ist es, ein Verfahren zu bereitzustellen, das den oben genannten Nachteilen bekannter Verfahren abhilft. Insbesondere soll ein Verfahren bereitgestellt werden, bei dem Ablagerungen durch hochsiedende organische Nebenbestandteile in den der ODH nachgeschalteten Apparaten vermeiden werden. Weiterhin soll ein Verfahren bereitgestellt werden, bei dem die mögliche Anreicherung organischer Peroxide vermieden wird. Weiterhin ist es Aufgabe der Erfindung, hohe Belastungen von Abwasser mit organischen Verbindungen in gelöster, emulgierter oder suspendierter Form, sowie den Anfall von mit organischen Verbindungen belasteten Abwässern zu verringern. Diese Aufgaben sollen gelöst werden, ohne dass es zu einer Beeinträchtigung der Katalysatoraktivität durch Spuren von organischen Lösungsmitteln in dem in die ODH rückgeführten Kreisgas kommt.

Gelöst wird die Aufgabe durch ein Verfahren zur Herstellung von Butadien aus n-Butenen mit den Schritten:

A) Bereitstellung eines n-Butene enthaltenden Einsatzgasstroms a1,

B) Einspeisung des n-Butene enthaltenden Einsatzgasstromes a1, eines sauerstoffhaltigen Gases und eines sauerstoffhaltigen Kreisgasstroms a2 in mindestens eine oxidative Dehydrierzone und oxidative Dehydrierung von n-Butenen zu Butadien, wobei ein Produktgasstrom b enthaltend Butadien, nicht umgesetzte n-Butene, Wasserdampf, Sauerstoff, leicht siedende Kohlenwasserstoffe, hochsiedende Nebenkomponenten, gegebenenfalls Kohlenstoffoxide und gegebenenfalls Inertgase erhalten wird,

Ca) Abkühlung des Produktgasstroms b und gegebenenfalls zumindest teilweise Abtrennung von hochsiedenden Nebenkomponenten und von Wasserdampf, wobei ein Produktgasstrom b' erhalten wird,

Cb) Kompression und Kühlung des Produktgasstroms b' in mindestens einer Kompressions- und Kühlungsstufe, wobei mindestens ein wässriger Kondensatstrom c1 und ein Gasstrom c2 enthaltend Butadien, n-Butene, Wasserdampf, Sauerstoff, leicht siedende Kohlenwasserstoffe, gegebenenfalls Kohlenstoffoxide und gegebenenfalls Inertgase erhalten wird,

Da) Absorption der $C_4$-Kohlenwasserstoffe umfassend Butadien und n-Butene in einem aromatischen Kohlenwasserstofflösungsmittel als Absorptionsmittel und Abtrennung von nicht kondensierbaren und leicht siedenden Gasbestandteilen umfassend Sauerstoff, leicht siedende Kohlenwasserstoffe, gegebenenfalls Kohlenstoffoxide, aromatisches Kohlenwasserstofflösungsmittel und gegebenenfalls Inertgase als Gasstrom d2 aus dem Gasstrom c2, wobei ein mit $C_4$-Kohlenwasserstoffen beladener Absorptionsmittelstrom und der Gasstrom d2 erhalten werden, und anschließende Desorption der $C_4$-Kohlenwasserstoffe aus dem beladenen Absorptionsmittelstrom, wobei ein $C_4$-Produktgasstrom d1 erhalten wird,

Db) zumindest teilweise Rückführung des Gasstroms d2 als Kreisgasstrom a2 in die oxidative Dehydrierzone,

dadurch gekennzeichnet, dass der Gehalt an aromatischem Kohlenwasserstofflösungsmittel im Kreisgasstrom a2 auf weniger als 0,2 Vol.-% begrenzt wird.

Die Bestimmung des Volumenanteils von Mesitylen und der weiteren Gasbestandteile erfolgt gaschromatographisch. Die Kalibrierung von Mesitylen erfolgt hierbei mittels eines externen Standards. Dafür wird ein vergasbares Lösungsmittel, beispielsweise m-Xylol, mit Mesitylen in einem bestimmten molaren Verhältnis in einem Lösungsmittel, wie zum Beispiel Aceton, gelöst. Unter der Annahme, dass beide Stoffe und das Lösemittel sich als ideale Gase verhalten, wird der Molanteil in Volumenanteile umgerechnet.

Die Gasprobe mit bekanntem Volumenanteil des vergasbaren Lösungsmittels wird über eine Probeschleife dem GC zugeführt. Die Probenschleife mit definiertem Volumen wird bei konstantem Druck und konstanter Temperatur betrieben, worauf sich aus den Flächen des Vergleichsstoffes und des Mesitylens ein externer Faktor ermitteln lässt. Dieser kann jetzt ins Verhältnis zum Mesitylen gesetzt werden.

Die weiteren Komponenten werden auf ähnliche Weise einzeln oder in Mischungen kalibriert. Hierbei werden sämtliche Komponenten wie ideale Gase behandelt. Dies gilt genauso für die Analyse der Gasströme im ODH-Prozess.

Es wurde gefunden, dass erhöhte Mengen an aromatischen Kohlenwasserstofflösungsmitteln in dem Reaktionsgasgemisch der Oxidehydrierung die Katalysatoraktivität beeinträchtigen. Die Menge an aromatischen Kohlenwasserstofflösungsmittel im Reaktionsgasgemisch hängt vor allem vom Anteil des aromatischen Kohlenwasserstofflösungsmittel im Kreisgas sowie vor allem vom Anteil des Kreisgases am Reaktionsgasgemisch ab.

Die Auswahl des Kühlmittels in der Abkühlstufe Ca) unterliegt keinen Einschränkungen. Jedoch wird in der Abkühlstufe Ca) bevorzugt ein organisches Lösungsmittel verwendet. Diese weisen im Allgemeinen ein sehr viel höheres Lösungsvermögen für die hochsiedenden Nebenprodukte, die in den dem ODH-Reaktor nachgelagerten Anlageteilen zu Ablagerungen und Verstopfungen führen können, auf als Wasser oder alkalisch-wässrige Lösungen. Bevorzugte als Abkühlmittel eingesetzte organische Lösungsmittel sind aromatische Kohlenwasserstoffe, besonders bevorzugt sind Toluol, o-Xylol, m-Xylol, p-Xylol, Mesitylen, alle möglichen Konstitutionsisomere von Mono-, Di- und Triethylbenzol sowie alle möglichen Konstitutionsisomere von Mono-, Di- und Triisopropylbenzol oder Gemische daraus. Bevorzugt sind aromatische Kohlenwasserstoffe mit einem Siedepunkt bei 1013,25 hPa von über 120 °C oder Gemische daraus. Speziell bevorzugt ist Mesitylen.

Das in der Abtrennstufe Da) verwendete Absorptionsmittel ist ein aromatisches Kohlenwasserstofflösungsmittel. Bevorzugt sind Toluol, o-Xylol, m-Xylol, p-Xylol, Mesitylen, alle möglichen Konstitutionsisomere von Mono-, Di- und Triethylbenzol sowie alle möglichen Konstitutionsisomere von Mono-, Di- und Triisopropylbenzol oder Gemische daraus. Bevorzugt sind aromatische Kohlenwasserstoffe mit einem Siedepunkt bei 1013,25 hPa von über 120 °C. Besonders bevorzugt ist Mesitylen. Insbesondere wird in der Abtrennstufe Da) das gleiche aromatische Kohlenwasserstofflösungsmittel eingesetzt wie in der vorhergehenden Abkühlstufe Ca), wenn in der Abkühlstufe Ca) ein organisches Lösungsmittel verwendet wird.

Durch Absorption der $C_4$-Kohlenwasserstoffe umfassend Butadien und n-Butene aus dem Gasstrom c2 in dem aromatischen Kohlenwasserstofflösungsmittel werden nicht kondensierbaren und leicht siedende Gasbestandteilen umfassend Sauerstoff, leicht siedende Kohlenwasserstoffe, gegebenenfalls Kohlenstoffoxide und gegebenenfalls Inertgase als Gasstrom d2 erhalten. Zumindest ein Teil dieses Gasstroms d2 wird als Kreisgasstrom a2 in die oxidative Dehydrierung (Schritt B)) zurückgeführt. Erfindungsgemäß beträgt der Gehalt an aromatischem Kohlenwasserstofflösungsmittel im Kreisgasstroms a2 weniger als 0,2 Vol.-%.

In einer Ausführungsform der Erfindung wird der Gehalt an aromatischem Kohlenwasserstofflösungsmittel im Kreisgasstrom a2 auf weniger als 1 Vol.-% dadurch begrenzt, dass die Abtrennstufe Da) bei Temperaturen bevorzugt unterhalb von 50 °C, besonders bevorzugt bei unterhalb von 40 °C und/oder bei einem höheren Druck als 5 bar absolut, besonders bevorzugt bei 10 bar absolut und höher betrieben wird. Zur Einstellung der Temperatur kann beispielsweise das in der Abtrennstufe Da) verwendete Absorptionsmittel vor Eintritt in die Abtrennstufe Da) auf eine niedrige Temperatur abgekühlt werden.

In einer weiteren Ausführungsform der Erfindung wird der Gehalt an aromatischem Kohlenwasserstofflösungsmittel im Kreisgasstrom a2 auf weniger als 1 Vol.-% dadurch begrenzt, dass der die Abtrennstufe Da) verlassende Gasstrom in einer weiteren Kolonne mit einem flüssigen Absorbens für das aromatische Kohlenwasserstofflösungsmittel in Kontakt gebracht wird. Das in dieser weiteren Kolonne verwendete Absorbens muss mit dem aromatischen Kohlenwasserstofflösungsmittel aus der Absorberkolonne der Abtrennstufe Da) mischbar sein und kann wahlweise auch das gleiche Lösungsmittel sein. Falls es sich bei dem in der weiteren Kolonne verwendeten Absorbens um das gleiche Lösungsmittel handelt, ist der Druck in dieser weiteren Kolonne höher als in der Absorberkolonne der Abtrennstufe Da), oder aber der dieser weiteren Kolonne zugeführte Absorbensstrom ist kälter als der in diese Kolonne eintretende Gasstrom, wodurch das in dem Gasstrom d2 enthaltene aromatische Kohlenwasserstofflösungsmittel zumindest teilweise abgetrennt wird.

In einer weiteren Ausführungsform der Erfindung wird der Gehalt an aromatischem Kohlenwasserstofflösungsmittel im Kreisgasstrom a2 auf weniger als 1 Vol.-% dadurch begrenzt, dass der der die Abtrennstufe Da) verlassende Gasstrom d2 mit einem festen Adsorbens in Kontakt gebracht wird, das das aromatische Kohlenwasserstofflösungsmittel adsorbiert. Dabei können Adsorbentien wie z.B. Aktivkohle verwendet werden, durch die der Strom d2 geleitet wird. Geeignete Adsorbentien sind beispielsweise in Ullmann's Encyclopedia of Industrial Chemistry (2011), 7, 209-210 und 3, 527-528 beschrieben.

In einer weiteren Ausführungsform der Erfindung wird der Gehalt an aromatischem Kohlenwasserstofflösungsmittel im Kreisgasstrom a2 auf weniger als 0,2 Vol.-% dadurch begrenzt, dass der die Abtrennstufe Da) verlassende Gasstrom d2 mit einem als Kondensator ausgeführtem Wärmetauscher in Kontakt gebracht wird, wobei durch Abkühlung das in dem Strom d2 enthaltene aromatischem Kohlenwasserstofflösungsmittel zumindest teilweise als Flüssigphase abgeschieden wird.

In einer weiteren Ausführungsform der Erfindung wird der Gehalt an aromatischem Kohlenwasserstofflösungsmittel im Kreisgasstrom a2 auf weniger als 1 Vol.-% durch eine thermische oder katalytische Nachverbrennung des aromatischen Kohlenwasserstofflösungsmittels begrenzt. Geeignete Katalysatoren für eine katalytische Nachverbrennung sind vielfach in der Literatur beschrieben worden, siehe zum Beispiel Prasad et al., Catal. Rev. Sci. Eng. 26 (1984), 1; Trimm, Appl. Catal. 7 (1983), 249; Arai et al., Catal. Today 26 (1995), 217; Centi, J. Mol. Catal. A 173 (2001), 287; Kirchnerova, Kor. J. Chem. Eng. 16 (1999), 427; Ciuparu et al., Catal. Rev. Sci. Eng. 44 (2002), 593 und die darin zitierte Literatur. Bei der Verbrennung werden zumindest Teile der brennbaren Bestandteile des Kreisgases in Anwesenheit von Sauerstoff oxidiert. Brennbare Bestandteile des Kreisgases sind beispielsweise CO und das aromatische Kohlenwasserstofflösungsmittel. Um die Brennbarkeit des Kreisgases zu erhöhen, kann es von Vorteil sein, dem Kreisgas weitere brennbare Bestandteile, wie zum Beispiel Methan, Ethan oder Wasserstoff zuzusetzen.

Die Verbrennung kann adiabatisch, autotherm oder isotherm erfolgen, zum Beispiel in einem Wirbelbett, im Hordenofen, im Festrohr- oder Rohrbündelreaktor oder im Plattenwärmetauscherreaktor. Die Strömung durch die Schüttung kann sowohl axial als auch radial orientiert werden. Monolith-Reaktoren können für Reaktionen, die wenig Katalysator benötigen, vorteilhaft als Adiabat-Reaktor eingesetzt werden.

Die Eintrittstemperatur des Kreisgases im Katalysatorbett beträgt typischerweise über 100 °C, bevorzugt über 200 °C. Um diese Temperaturen zu erreichen, kann das Kreisgas vorgewärmt werden.

In einer weiteren Ausführungsform der Erfindung wird der Gehalt an aromatischem Kohlenwasserstofflösungsmittel im Kreisgasstrom a2 auf weniger als 0,2 Vol.-% dadurch begrenzt, dass in oder nach der im Absorptionsschritt Da) eingesetzten Absorptionskolonne eine oder mehrere Vorrichtungen, beispielsweise ein Demister oder Tropfenabscheider, vorgesehen werden, die den Mitriss von flüssigen Bestandteilen aus der Absorptionskolonne in den Gasstrom d2 verringern. Geeignet sind alle Vorrichtungen, die den Anteil an flüssigen Bestandteilen im Gasstrom d2 verringern. Im Allgemeinen versteht man unter Demistern oder Tropfenabscheidern Vorrichtungen zur Abscheidung feinster Flüssigkeitströpfchen aus Gasen, Dämpfen oder Nebeln, allgemein Aerosolen. In Kolonnen kann der Flüssigkeitsmitriss durch Demister oder Tropfenabscheider vermindert werden. Demister oder Tropfenabscheider können beispielsweise aus Drahtgestrickpackungen, Lamellenabscheidern oder Schüttungen aus Füllkörpern mit großer innerer Oberfläche bestehen. In der Regel werden als Werkstoffe Stähle, Chromnickelstähle, Aluminium, Kupfer, Nickel, Polypropylen, Polytetrafluorethylen und dergleichen benutzt. Der Abscheidegrad verringert sich mit kleiner werdenden Tröpfchendurchmessern. Demister können zu den Koaleszenzabscheidern gezählt werden. Demister sind unter anderem in den Anmeldungen US 3,890,123 und US 4,141,706 und den darin zitierten Schriften beschrieben. Der Demister oder Tropfenabscheider kann sich sowohl innerhalb der Absorptionskolonne oder den Absorptionskolonnen befinden, oder diesen nachgeschaltet sein.

Bevorzugt beträgt der Gehalt des Kreisgasstroms a2 an aromatischem Kohlenwasserstofflösungsmittel weniger als 0,2 Vol.-%, insbesondere weniger als 0,1 Vol.-%.

Bevorzugt umfasst das erfindungsgemäße Verfahren noch folgende weitere Verfahrensschritte:

E) Auftrennung des $C_4$-Produktstroms d1 durch Extraktivdestillation mit einem für Butadien selektiven Lösungsmittel in einen Butadien und das selektive Lösungsmittel enthaltenden Stoffstrom e1 und einen n-Butene enthaltenden Stoffstrom e2;

F) Destillation des Butadien und das selektive Lösungsmittel enthaltenden Stoffstroms e2 in einen das selektiven Lösungsmittel enthaltenden Stoffstrom f1 und einen Butadien enthaltenden Stoffstrom f2.

Nachstehende Ausführungsformen sind bevorzugte bzw. besonders bevorzugte Varianten des erfindungsgemäßen Verfahrens:

Der Stufe Ca) kann mindestens eine Kühlstufe vorgeschaltet werden, in der der Produktgasstrom b durch indirekte Kühlung in einem Wärmetauscher gekühlt wird.

Die Stufe Ca) kann mehrstufig in Stufen Ca1) bis Can), bevorzugt zweistufig in zwei Stufen Ca1) und Ca2) durchgeführt werden. Dabei wird besonders bevorzugt zumindest ein Teil des Kühlmittels nach Durchlaufen der zweiten Stufe Ca2) als Abkühlmittel der ersten Stufe Ca1) zugeführt.

Die Stufe Cb) umfasst im Allgemeinen mindestens eine Kompressionsstufe Cba) und mindestens eine Abkühlstufe Cbb). Bevorzugt wird in der mindestens einen Abkühlstufe Cbb) das in der Kompressionsstufe Cba) komprimierte Gas mit einem Abkühlmittel in Kontakt gebracht. Besonders bevorzugt enthält das Abkühlmittel der Abkühlstufe Cbb) das gleiche organische Lösungsmittel, das in der Stufe Ca) als Abkühlmittel verwendet wird, wenn in der Abkühlstufe Ca) ein organisches Lösungsmittel verwendet wird. In einer insbesonders bevorzugten Variante wird zumindest ein Teil dieses Abkühlmittels nach Durchlaufen der mindestens einen Abkühlstufe Cbb) als Abkühlmittel der Stufe Ca) zugeführt. Alternativ kann die Abkühlstufe Cbb) aus Wärmetauschern bestehen.

Bevorzugt umfasst die Stufe Cb) mehrere Kompressionsstufen Cba1) bis Cban) und Abkühlstufen Cbb1) bis Cbbn), beispielsweise vier Kompressionsstufen Cba1) bis Cba4) und vier Abkühlstufen Cbb1) bis Cbb4).

Bevorzugt umfasst Schritt Da) die Schritte Daa) bis Dac):

Daa) Absorption der $C_4$-Kohlenwasserstoffe umfassend Butadien und n-Butene in einem aromatischen Kohlenwasserstofflösungsmittel als Absorptionsmittel, wobei ein mit $C_4$-Kohlenwasserstoffen beladener Absorptionsmittelstrom und der Gasstrom d2 erhalten werden,

Dab) Entfernung von Sauerstoff aus dem mit $C_4$-Kohlenwasserstoffen beladenen Absorptionsmittelstrom aus Schritt Daa) durch Strippung mit einem nicht kondensierbaren Gasstrom, und

Dac) Desorption der $C_4$-Kohlenwasserstoffe aus dem beladenen Absorptionsmittelstrom, wobei ein $C_4$-Produktgasstrom d1 erhalten wird, der im Wesentlichen aus $C_4$-Kohlenwasserstoffen besteht und weniger als 100 ppm Sauerstoff umfasst.

Ausführungsformen des erfindungsgemäßen Verfahrens werden im Folgenden detailliert beschrieben.

Als Einsatzgasstrom a1 können reine n-Butene (1-Buten und/oder cis-2-Buten und/oder trans-2-Buten), aber auch Butene enthaltende Gasgemische eingesetzt werden. Ein solches Gasgemisch kann beispielsweise durch nicht-oxidative Dehydrierung von n-Butan erhalten werden. Es kann auch eine Fraktion eingesetzt werden, die als Hauptbestandteil n-Butene enthält und aus der $C_4$-Fraktion des Naphtha-Crackens durch Abtrennung von Butadien und iso-Buten erhalten wurde. Des Weiteren können auch Gasgemische als Einsatzgasstrom eingesetzt werden, die reines 1-Buten, cis-2-Buten, trans-2-Buten oder Mischungen daraus umfassen, und die durch Dimerisierung von Ethylen erhalten wurden. Ferner können als Einsatzgasstrom n-Butene enthaltende Gasgemische eingesetzt werden, die durch katalytisches Wirbelschichtkracken (Fluid Catalytic Cracking, FCC) erhalten wurden.

In einer Ausführungsform des erfindungsgemäßen Verfahrens wird der n-Butene enthaltende Einsatzgasstrom durch nicht-oxidative Dehydrierung von n-Butan erhalten. Durch die Kopplung einer nicht-oxidativen katalytischen Dehydrierung mit der oxidativen Dehydrierung der gebildeten n-Butene kann eine hohe Ausbeute an Butadien, bezogen auf eingesetztes n-Butan, erhalten werden. Bei der nicht-oxidativen katalytischen n-Butan-Dehydrierung wird ein Gasgemisch erhalten, das neben Butadien 1-Buten, 2-Buten und nicht umgesetztem n-Butan Nebenbestandteile enthält. Übliche Nebenbestandteile sind Wasserstoff, Wasserdampf, Stickstoff, CO und $CO_2$, Methan, Ethan, Ethen, Propan und Propen. Die Zusammensetzung des die erste Dehydrierzone verlassenden Gasgemischs kann abhängig von der Fahrweise der Dehydrierung stark variieren. So weist bei Durchführung der Dehydrierung unter Einspeisung von Sauerstoff und zusätzlichem Wasserstoff das Produktgasgemisch einen vergleichsweise hohen Gehalt an Wasserdampf und Kohlenstoffoxiden auf. Bei Fahrweisen ohne Einspeisung von Sauerstoff weist das Produktgasgemisch der nichtoxidativen Dehydrierung einen vergleichsweise hohen Gehalt an Wasserstoff auf.

In Schritt B) wird das Reaktionsgasgemisch enthaltend den n-Butene enthaltenden Einsatzgasstrom a1, ein sauerstoffhaltiges Gas, einen sauerstoffhaltigen Kreisgasstrom a2 und gegebenenfalls weitere Komponenten in mindestens eine Dehydrierzone (den ODH-Reaktor) eingespeist und die in dem Gasgemisch enthaltenen Butene in Gegenwart eines Oxidehydrierungskatalysators oxidativ zu Butadien dehydriert.

Für die Oxidehydrierung geeignete Katalysatoren basieren im Allgemeinen auf einem Mo-Bi-O-haltigen Multimetalloxidsystem, das in der Regel zusätzlich Eisen enthält. Im Allgemeinen enthält das Katalysatorsystem noch weitere zusätzliche Komponenten, wie beispielsweise Kalium, Cäsium, Magnesium, Zirkon, Chrom, Nickel, Cobalt, Cadmium, Zinn, Blei, Germanium, Lanthan, Mangan, Wolfram, Phosphor, Cer, Aluminium oder Silizium. Auch eisenhaltige Ferrite wurden als Katalysatoren vorgeschlagen.

In einer bevorzugten Ausführungsform enthält das Multimetalloxid Cobalt und/oder Nickel. In einer weiteren bevorzugten Ausführungsform enthält das Multimetalloxid Chrom. In einer weiteren bevorzugten Ausführungsform enthält das Multimetalloxid Mangan.

Beispiele für Mo-Bi-Fe-O-haltige Multimetalloxide sind Mo-Bi-Fe-Cr-O- oder Mo-Bi-Fe-Zr-O-haltige Multimetalloxide. Bevorzugte Systeme sind beispielsweise beschrieben in US 4,547,615 ($Mo_{12}BiFe_{0,1}Ni_8ZrCr_3K_{0,2}O_x$ und $Mo_{12}BiFe_{0,1}Ni_8AlCr_3K_{0,2}O_x$), US 4,424,141 ($Mo_{12}BiFe_3Co_{4,5}Ni_{2,5}P_{0,5}K_{0,1}O_x$ + $SiO_2$), DE-A 25 30 959 ($Mo_{12}BiFe_3Co_{4,5}Ni_{2,5}Cr_{0,5}K_{0,1}O_x$, $Mo_{13,75}BiFe_3Co_{4,5}Ni_{2,5}Ge_{0,5}K_{0,8}O_x$, $Mo_{12}BiFe_3Co_{4,5}Ni_{2,5}Mn_{0,5}K_{0,1}O_x$ und $Mo_{12}BiFe_3CO_{4,5}Ni_{2,5}La_{0,5}K_{0,1}O_x$), US 3,911,039 ($Mo_{12}BiFe_3Co_{4,5}Ni_{2,5}Sn_{0,5}K_{0,1}O_x$), DE-A 25 30 959 und DE-A 24 47 825 ($Mo_{12}BiFe_3Co_{4,5}Ni_{2,5}W_{0,5}K_{0,1}O_x$).

Geeignete Multimetalloxide und deren Herstellung sind weiterhin beschrieben in US 4,423,281 ($Mo_{12}BiNi_8Pb_{0,5}Cr_3K_{0,2}O_x$ und $Mo_{12}Bi_bNi_7Al_3Cr_{0,5}K_{0,5}O_x$), US 4,336,409 ($Mo_{12}BiNi_6Cd_2Cr_3P_{0,5}O_x$), DE-A 26 00 128 ($Mo_{12}BiNi_{0,5}Cr_3P_{0,5}Mg_{7,5}K_{0,1}O_x$ + $SiO_2$) und DE-A 24 40 329 ($Mo_{12}BiCo_{4,5}Ni_{2,5}Cr_3P_{0,5}K_{0,1}O_x$).

Besonders bevorzugte katalytisch aktive, Molybdän und mindestens ein weiteres Metall enthaltende Multimetalloxide weisen die allgemeine Formel (Ia) auf:

$$Mo_{12}Bi_aFe_bCo_cNi_dCr_eX^1_fX^2_gO_y \qquad (Ia),$$

mit

$X^1 =$   Si, Mn und/oder Al,
$X^2 =$   Li, Na, K, Cs und/oder Rb,

$$0,2 \leq a \leq 1,$$

$$0,5 \leq b \leq 10,$$

$$0 \leq c \leq 10,$$

$$0 \leq d \leq 10,$$

$$2 \leq c + d \leq 10$$

$$0 \leq e \leq 2,$$

$$0 \leq f \leq 10$$

$$0 \leq g \leq 0,5$$

y = eine Zahl, die unter der Voraussetzung der Ladungsneutralität durch die Wertigkeit und Häufigkeit der von Sauerstoff verschiedenen Elemente in (Ia) bestimmt wird.

Bevorzugt sind Katalysatoren, deren katalytisch aktive Oxidmasse von den beiden Metallen Co und Ni nur Co aufweist (d = 0). Bevorzugte ist $X^1$ Si und/oder Mn und $X^2$ ist vorzugsweise K, Na und/oder Cs, besonders bevorzugt ist $X^2$ = K.

Das molekularen Sauerstoff enthaltende Gas enthält im Allgemeinen mehr als 10 Vol.-%, vorzugsweise mehr als 15 Vol.-% und noch mehr bevorzugt mehr als 20 Vol.-% molekularen Sauerstoff. Bevorzugt ist es Luft. Die Obergrenze für den Gehalt an molekularem Sauerstoff beträgt im Allgemeinen 50 Vol.-% oder weniger, vorzugsweise 30 Vol.-% oder weniger und noch mehr bevorzugt 25 Vol.-% oder weniger. Darüber hinaus können in dem molekularen Sauerstoff enthaltenden Gas beliebige Inertgase enthalten sein. Als mögliche Inertgase können Stickstoff, Argon, Neon, Helium, CO, $CO_2$ und Wasser genannt werden. Die Menge an Inertgasen beträgt für Stickstoff im Allgemeinen 90 Vol.-% oder weniger, vorzugsweise 85 Vol.-% oder weniger und noch mehr bevorzugt 80 Vol.-% oder weniger. Im Falle anderer Bestandteile als Stickstoff beträgt sie im Allgemeinen 10 Vol.-% oder weniger, vorzugsweise 1 Vol.-% oder weniger.

Zur Durchführung der oxidativen Dehydrierung bei Vollumsatz von n-Butenen ist ein Gasgemisch bevorzugt, welches ein molares Sauerstoff: n-Butene-Verhältnis von mindestens 0,5 aufweist. Bevorzugt wird bei einem Sauerstoff: n-Butene-Verhältnis von 0,55 bis 10 gearbeitet. Zur Einstellung dieses Wertes kann der Eingangsgasstrom mit Sauerstoff oder mindestens einem sauerstoffhaltigem Gas, beispielsweise Luft, und gegebenenfalls zusätzlichem Inertgas oder Wasserdampf vermischt werden. Das erhaltene sauerstoffhaltige Gasgemisch wird dann der Oxidehydrierung zugeführt.

Ferner können zusammen im Reaktionsgasgemisch auch inerte Gase wie Stickstoff und weiterhin Wasser (als Wasserdampf) enthalten sein. Stickstoff kann zur Einstellung der Sauerstoffkonzentration und zur Verhinderung der Ausbildung eines explosionsfähigen Gasgemischs dienen, das gleiche gilt für Wasserdampf. Wasserdampf dient ferner zur Kontrolle des Verkokens des Katalysators und zur Abfuhr der Reaktionswärme.

Die Reaktionstemperatur der Oxidehydrierung wird im Allgemeinen durch ein Wärmeaustauschmittel, welches sich um die Reaktionsrohre herum befindet, kontrolliert. Als solche flüssige Wärmeaustauschmittel kommen z.B. Schmelzen von Salzen wie Kaliumnitrat, Kaliumnitrit, Natriumnitrit und/oder Natriumnitrat sowie Schmelzen von Metallen wie Natrium, Quecksilber und Legierungen verschiedener Metalle in Betracht. Aber auch ionische Flüssigkeiten oder Wärmeträgeröle sind einsetzbar. Die Temperatur des Wärmeaustauschmittels liegt zwischen 220 bis 490 °C und bevorzugt zwischen 300 bis 450 °C und besonders bevorzugt zwischen 350 und 420 °C.

Aufgrund der Exothermie der ablaufenden Reaktionen kann die Temperatur in bestimmten Abschnitten des Reaktorinneren während der Reaktion höher liegen als diejenige des Wärmeaustauschmittels und es bildet sich ein so genannter Hotspot aus. Die Lage und Höhe des Hotspots ist durch die Reaktionsbedingungen festgelegt, aber sie kann auch durch das Verdünnungsverhältnis der Katalysatorschicht oder den Durchfluss an Mischgas reguliert werden. Die Differenz zwischen Hotspot-Temperatur und der Temperatur des Wärmeaustauschmittels liegt im Allgemeinen zwischen 1-150 °C, bevorzugt zwischen 10-100 °C und besonders bevorzugt zwischen 20-80 °C. Die Temperatur am Ende des Katalysatorbettes liegt im Allgemeinen zwischen 0-100 °C, vorzugsweise zwischen 0,1-50 °C, besonders bevorzugt zwischen 1-25 °C oberhalb der Temperatur des Wärmeaustauschmittels.

Die Oxidehydrierung kann in allen aus dem Stand der Technik bekannten Festbettreaktoren durchgeführt werden, wie beispielsweise im Hordenofen, im Festbettrohr- oder Rohrbündelreaktor oder im Plattenwärmetauscherreaktor. Ein Rohrbündelreaktor ist bevorzugt.

Vorzugsweise wird die oxidative Dehydrierung in Festbettrohrreaktoren oder Festbettrohrbündelreaktoren durchgeführt. Die Reaktionsrohre werden (ebenso wie die anderen Elemente des Rohrbündelreaktors) in der Regel aus Stahl gefertigt. Die Wanddicke der Reaktionsrohre beträgt typischerweise 1 bis 3 mm. Ihr Innendurchmesser liegt in der Regel (einheitlich) bei 10 bis 50 mm oder bei 15 bis 40 mm, häufig bei 20 bis 30 mm. Die im Rohrbündelreaktor untergebrachte Anzahl an Reaktionsrohren beläuft sich in der Regel wenigstens auf 1000, oder 3000, oder 5000, vorzugsweise auf wenigstens 10 000. Häufig beträgt die Anzahl der im Rohrbündelreaktor untergebrachten Reaktionsrohre 15 000 bis 30 000 bzw. bis 40 000 oder bis 50 000. Die Länge der Reaktionsrohre erstreckt sich im Normalfall auf wenige Meter, typisch ist eine Reaktionsrohrlänge im Bereich von 1 bis 8 m, häufig 2 bis 7 m, vielfach 2,5 bis 6 m.

Weiterhin kann die Katalysatorschicht, die im ODH-Reaktor eingerichtet ist, aus einer einzelnen Schicht oder aus 2 oder mehr Schichten bestehen. Diese Schichten können aus reinem Katalysator bestehen oder mit einem Material verdünnt sein, das nicht mit dem Einsatzgasstrom oder Komponenten aus dem Produktgas der Reaktion reagiert.

Weiterhin können die Katalysatorschichten aus Vollmaterial oder geträgerten Schalenkatalysatoren bestehen.

Der die oxidative Dehydrierung verlassende Produktgasstrom enthält neben Butadien im Allgemeinen noch nicht umgesetztes 1-Buten und 2-Buten, Sauerstoff sowie Wasserdampf. Als Nebenkomponenten enthält er weiterhin im Allgemeinen Kohlenmonoxid, Kohlendioxid, Inertgase (hauptsächlich Stickstoff), leichtsiedende Kohlenwasserstoffe wie Methan, Ethan, Ethen, Propan und Propen, Butan und iso-Butan, gegebenenfalls Wasserstoff sowie gegebenenfalls sauerstoffhaltige Kohlenwasserstoffe, sogenannte Oxygenate. Oxygenate können beispielsweise Formaldehyd, Furan, Essigsäure, Maleinsäureanhydrid, Ameisensäure, Methacrolein, Methacrylsäure, Crotonaldehyd, Crotonsäure, Propionsäure, Acrylsäure, Methylvinylketon, Benzaldehyd, Benzoesäure, Phthalsäureanhydrid, Fluorenon, Anthrachinon und Butyraldehyd sein.

Der Produktgasstrom am Reaktorausgang ist durch eine Temperatur nahe der Temperatur am Ende des Katalysatorbetts charakterisiert. Der Produktgasstrom wird dann auf eine Temperatur von 150 bis 400 °C, bevorzugt 160 bis 300 °C, besonders bevorzugt 170 bis 250 °C gebracht. Es ist möglich, die Leitung, durch die der Produktgasstrom fließt, um die Temperatur im gewünschten Bereich zu halten, zu isolieren oder einen Wärmetauscher einzusetzen. Dieses Wärmetauschersystem ist beliebig, solange mit diesem System die Temperatur des Produktgases auf dem gewünschten Niveau gehalten werden kann. Als Beispiel für Wärmetauscher können Spiralwärmetauscher, Plattenwärmetauscher, Doppelrohrwärmetauscher, Multirohrwärmetauscher, Kessel-Spiralwärmetauscher, Kessel-Mantelwärmetauscher, Flüssigkeit-Flüssigkeit-Kontakt-Wärmetauscher, Luft-Wärmetauscher, Direktkontaktwärmetauscher sowie Rippenrohrwärmetauscher genannt werden. Da, während die Temperatur des Produktgases auf die gewünschte Temperatur eingestellt wird, ein Teil der hochsiedenden Nebenprodukte, die im Produktgas enthalten sind, ausfallen kann, sollte daher das Wärmetauschersystem vorzugsweise zwei oder mehr Wärmetauscher aufweisen. Falls dabei zwei oder mehr vorgesehene Wärmetauscher parallel angeordnet sind, und so eine verteilte Kühlung des gewonnenen Produktgases in den Wärmetauschern ermöglicht wird, nimmt die Menge an hochsiedenden Nebenprodukten, die sich in den Wärmetauschern ablagern, ab und so kann ihre Betriebsdauer verlängert werden. Als Alternative zu der oben genannten Methode können die zwei oder mehr vorgesehenen Wärmetauscher parallel angeordnet sein. Das Produktgas wird einem oder mehreren, nicht aber allen, Wärmetauschern zugeführt, welche nach einer gewissen Betriebsdauer von anderen Wärmetauschern abgelöst werden. Bei dieser Methode kann die Kühlung fortgesetzt werden, ein Teil der Reaktionswärme zurückgewonnen und parallel dazu können die in einem der Wärmetauscher abgelagerten hochsiedenden Nebenprodukte entfernt werden. Als ein oben genanntes organisches Lösungsmittel kann ein Lösungsmittel, solange es in der Lage ist, die hochsiedenden Nebenprodukte aufzulösen, verwendet werden. Beispiele sind aromatische Kohlenwasserstofflösungsmittel, wie z.B. Toluol und Xylole, sowie alkalische wässriges Lösungsmittel, wie z.B. die wässrige Lösung von Natriumhydroxid.

Anschließend wird aus dem Produktgasstrom durch Abkühlung und Kompression ein Großteil der hochsiedenden Nebenkomponenten und des Wassers abgetrennt. Diese Stufe wird nachfolgend auch als Quench bezeichnet. Dieser Quench kann aus nur einer Stufe oder aus mehreren Stufen bestehen. Die Abkühlung kann durch Inkontaktbringen mit einem Kühlmittel, bevorzugt einem organischen Lösungsmittel erfolgen. Als Kühlmedium werden organische Lösungsmittel, bevorzugt aromatische Kohlenwasserstoffe, besonders bevorzugt Toluol, o-Xylol, m-Xylol, p-Xylol, Mesitylen, alle möglichen Konstitutionsisomere von Mono-, Di- und Triethylbenzol sowie alle möglichen Konstitutionsisomere von Mono-, Di- und Triisopropylbenzol oder Gemische daraus, verwendet. Bevorzugt sind weiterhin aromatische Kohlenwasserstoffe mit einem Siedepunkt bei 1013,25 hPa von über 120 °C oder Gemische daraus.

Bevorzugt ist ein zweistufiger Quench, d. h. die Stufe Ca umfasst zwei Abkühlstufen Ca1) und Ca2), in denen der Produktgasstrom b mit dem organischen Lösungsmittel in Kontakt gebracht wird.

Im Allgemeinen hat das Produktgas, je nach Vorliegen und Temperaturniveau eines Wärmetauschers vor dem Quench, eine Temperatur von 100-440 °C. Das Produktgas wird in der 1. Quenchstufe mit dem Kühlmedium in Kontakt gebracht. Hierbei kann das Kühlmedium durch eine Düse eingebracht werden, um eine möglichst effiziente Durchmischung mit dem Produktgas zu erreichen. Zum gleichen Zweck können in der Quenchstufe Einbauten, wie zum Beispiel weitere Düsen, eingebracht werden, die das Produktgas und das Kühlmedium gemeinsam passieren. Der Kühlmitteleinlass in den Quench ist so ausgelegt, dass ein Verstopfen durch Ablagerungen im Bereich des Kühlmitteleinlasses minimiert wird.

Im Allgemeinen wird das Produktgas in der ersten Quenchstufe auf 5-180 °C, vorzugsweise auf 30-130 °C und noch mehr bevorzugt auf 60-110 °C gekühlt. Die Temperatur des Kühlmittelmediums am Einlass kann im Allgemeinen 25-200 °C, bevorzugt 40-120 °C, insbesondere bevorzugt 50-90 °C betragen. Der Druck in der ersten Quenchstufe ist nicht besonders eingeschränkt, beträgt aber im Allgemeinen 0,01-4 bar (ü), bevorzugt 0,1-2 bar (ü) und besonders bevorzugt 0,2-1 bar (ü). Wenn größere Mengen hochsiedende Nebenprodukte im Produktgas vorhanden sind, kann es leicht zur Polymerisation von hochsiedenden Nebenprodukten und zu Ablagerungen von Feststoffen, die durch hochsiedende Nebenprodukte in diesem Verfahrensabschnitt verursacht werden, kommen. Im Allgemeinen ist die Quenchstufe als Kühlturm ausgestaltet. Das im Kühlturm eingesetzte Kühlmedium wird häufig zirkulierend eingesetzt. Der Kreislaufstrom des Kühlmediums in Liter pro Stunde, bezogen auf den Massenstrom an Butadien in Gramm pro Stunde, kann im Allgemeinen 0,0001-5 l/g, bevorzugt 0,001-1 l/g und besonders bevorzugt 0,002-0.2 l/g betragen.

Die Temperatur des Kühlmediums im Sumpf kann im Allgemeinen 27-210 °C, bevorzugt 45-130 °C, insbesondere bevorzugt 55-95 °C betragen. Da die Beladung des Kühlmediums mit Nebenkomponenten im Laufe der Zeit zunimmt, kann ein Teil des beladenen Kühlmediums aus dem Umlauf als Purgestrom abgezogen und die Umlaufmenge durch Zugabe von unbeladenem Kühlmedium konstant gehalten werden. Das Verhältnis von Ablaufmenge und Zugabemenge hängt von der Dampfbeladung des Produktgases und der Produktgastemperatur am Ende der erste Quenchstufe ab.

Je nach Temperatur, Druck und Wassergehalt des Produktgases kann es in der ersten Quenchstufe zur Kondensation von Wasser kommen. In diesem Falle kann sich eine zusätzliche wässrige Phase bilden, welche zusätzlich wasserlösliche Nebenkomponenten enthalten kann. Diese kann dann im Sumpf der Quenchstufe abgezogen werden. Bevorzugt ist ein Betrieb, in dem sich in der ersten Quenchstufe keine wässrige Phase ausbildet.

Der abgekühlte und eventuell an Nebenkomponenten abgereicherte Produktgasstrom kann nun einer zweiten Quenchstufe zugeführt werden. In dieser kann er nun erneut mit einem Kühlmedium in Kontakt gebracht werden.

Die Wahl des Kühlmittels ist nicht besonders eingeschränkt. Als Kühlmedium werden bevorzugt organische Lösungsmittel, besonders bevorzugt aromatische Kohlenwasserstoffe, insbesondere Toluol, o-Xylol, m-Xylol, p-Xylol, Mesitylen, alle möglichen Konstitutionsisomere von Mono-, Di- und Triethylbenzol sowie alle möglichen Konstitutionsisomere von Mono-, Di- und Triisopropylbenzol oder Gemische daraus, verwendet. Bevorzugt sind weiterhin aromatische Kohlenwasserstoffe mit einem Siedepunkt bei 1013,25 hPa von über 120 °C oder Gemische daraus.

Im Allgemeinen wird das Produktgas bis zum Gasausgang der zweiten Quenchstufe auf 5 bis 100 °C, vorzugsweise auf 15 bis 85 °C und noch mehr bevorzugt auf 30 bis 70 °C gekühlt. Das Kühlmittel kann im Gegenstrom zum Produktgas zugeführt werden. In diesem Fall kann die Temperatur des Kühlmittelmediums am Kühlmitteleinlass 5 bis 100 °C, bevorzugt 15 bis 85 °C, insbesondere bevorzugt 30 bis 70 °C betragen. Der Druck in der zweiten Quenchstufe ist nicht besonders eingeschränkt, beträgt aber im Allgemeinen 0,01 bis 4 bar (ü), bevorzugt 0,1 bis 2 bar (ü) und besonders bevorzugt 0,2 bis 1 bar (ü). Die zweite Quenchstufe ist bevorzugt als Kühlturm ausgestaltet. Das im Kühlturm eingesetzte Kühlmedium wird häufig zirkulierend eingesetzt. Der Kreislaufstrom des Kühlmediums in Liter pro Stunde, bezogen auf den Massenstrom an Butadien in Gramm pro Stunde, kann im Allgemeinen 0,0001 bis 5 l/g, bevorzugt 0,3001 bis 1 l/g und besonders bevorzugt 0,002 bis 0,2 l/g betragen.

Je nach Temperatur, Druck und Wassergehalt des Produktgases kann es in der zweiten Quenchstufe zur Kondensation von Wasser kommen. In diesem Falle kann sich eine zusätzliche wässrige Phase bilden, welche zusätzlich wasserlösliche Nebenkomponenten enthalten kann. Diese kann dann im Sumpf der Quenchstufe abgezogen werden. Die Temperatur des Kühlmediums im Sumpf kann im Allgemeinen 20 bis 210 °C, bevorzugt 35 bis 120 °C, insbesondere bevorzugt 45 bis 85 °C betragen. Da die Beladung des Kühlmediums mit Nebenkomponenten im Laufe der Zeit zunimmt, kann ein Teil des beladenen Kühlmediums als Purgestrom aus dem Umlauf abgezogen werden, und die Umlaufmenge durch Zugabe von unbeladenem Kühlmedium konstant gehalten werden.

Um einen möglichst guten Kontakt von Produktgas und Kühlmedium zu erreichen, können Einbauten in der zweiten Quenchstufe vorhanden sein. Solche Einbauten umfassen zum Beispiel Glocken-, Zentrifugal- und/oder Siebböden, Kolonnen mit strukturierten Packungen, z.B. Blechpackungen mit einer spezifischen Oberfläche von 100 bis 1000 m$^2$/m$^3$ wie Mellapak® 250 Y, und Füllkörperkolonnen.

Die Kühlmittel-Umläufe der beiden Quenchstufen können sowohl voneinander getrennt als auch miteinander verbunden sein. So kann beispielsweise der Strom dem Strom zugeführt werden oder diesen ersetzen. Die gewünschte Temperatur der Umlaufströme kann über geeignete Wärmetauscher eingestellt werden.

In einer bevorzugten Ausführungsform der Erfindung wird also die Abkühlstufe Ca) zweistufig durchgeführt, wobei das mit Nebenkomponenten beladene Kühlmittel der zweiten Stufe Ca2) in die erste Stufe Ca1) geführt wird. Das der zweiten Stufe Ca2) entnommene Kühlmittel enthält weniger Nebenkomponenten als das der ersten Stufe Ca1) entnommene Kühlmittel.

Um den Mitriss von flüssigen Bestandteilen aus dem Quench in die Abgasleitung zu minimieren, können geeignete bauliche Maßnahmen, wie zum Beispiel der Einbau eines Demisters, getroffen werden. Weiterhin können hochsiedende Substanzen, welche im Quench nicht vom Produktgas abgetrennt werden durch weitere bauliche Maßnahmen, wie beispielsweise weitere Gaswäschen, aus dem Produktgas entfernt werden.

Es wird ein Gasstrom erhalten, in der n-Butan, 1-Buten, 2-Butene, Butadien, gegebenenfalls Sauerstoff, Wasserstoff, Wasserdampf, in geringen Mengen Methan, Ethan, Ethen, Propan und Propen, iso-Butan, Kohlenstoffoxide, Inertgase und Teile des im Quench verwendeten Kühlmittel enthält. Weiterhin können in diesem Gasstrom Spuren von hochsiedenden Komponenten verbleiben, welche im Quench nicht quantitativ abgetrennt wurden. Unter solche hochsiedenden Komponenten fallen beispielsweise Methylvinylketon, Methylethylketon, Crotonaldehyd, Acrylsäure, Propionsäure, Maleinsäureanhydrid, Ethylbenzol, Styrol, Furanon, Benzoesäure, Benza-Idehyd, Fluorenon und Anthrachinon. Weiterhin kann dieser Gasstrom Formaldehyd, Methacrolein und/oder Furan enthalten.

Anschließend wird der Gasstrom b' aus dem Abkühlschritt Ca), der an hochsiedenden Nebenkomponenten abgereichert ist, im Schritt Cb) in mindestens einer Kompressionsstufe Cba) und bevorzugt in mindestens einer Abkühlstufe Cbb) abgekühlt.

Der Produktgasstrom aus dem Quench wird in mindestens einer Kompressionsstufe komprimiert und nachfolgend in dem Kühlapparat weiter abgekühlt, wobei mindestens ein Kondensatstrom enthaltend Wasser entsteht. Wenn im Quench ein von Wasser verschiedenes Kühlmittel benutzt wird, kann weiterhin das im Quench verwendete Kühlmittel auskondensieren und gegebenenfalls eine separate Phase bilden. Es verbleibt ein Gasstrom enthaltend Butadien, 1-Buten, 2-Butene, Sauerstoff, Wasserdampf, gegebenenfalls leichtsiedende Kohlenwasserstoffe wie Methan, Ethan, Ethen, Propan und Propen, Butan und iso-Butan, gegebenenfalls Kohlenstoffoxide und gegebenenfalls Inertgase. Weiterhin kann dieser Produktgasstrom noch Spuren von hochsiedenden Komponenten enthalten.

Die Kompression und Kühlung des Gasstroms kann ein- oder mehrstufig (n-stufig) erfolgen. Im Allgemeinen wird insgesamt von einem Druck im Bereich von 1,0 bis 4,0 bar (absolut) auf einen Druck im Bereich von 3,5 bis 20 bar (absolut) komprimiert. Nach jeder Kompressionsstufe folgt eine Abkühlstufe, in der der Gasstrom auf eine Temperatur im Bereich von 15 bis 60 °C abgekühlt wird. Die Abkühlung erfolgt bevorzugt durch Inkontaktbringen mit einem organischen Lösungsmittel als Abkühlmittel. Alternativ können auch Wärmetauscher eingesetzt werden. Der Kondensatstrom kann somit bei mehrstufiger Kompression auch mehrere Ströme umfassen. Der Kondensatstrom besteht zu großen Teilen aus Wasser (wässrige Phase) und gegebenenfalls dem im Quench verwendeten Kühlmittel (organische Phase). Beide Ströme (wässrige und organische Phase) können daneben in geringem Umfang Nebenkomponenten wie Leichtsieder, $C_4$-Kohlenwasserstoffe, Oxygenate und Kohlenstoffoxide enthalten.

Um den Strom zu kühlen und/oder um weitere Nebenkomponenten aus dem Strom zu entfernen, kann das kondensierte Quench-Kühlmittel in einem Wärmetauscher abgekühlt und als Kühlmittel in den Apparat rückgeführt werden. Da die Beladung dieses Kühlmediums mit Nebenkomponenten im Laufe der Zeit zunimmt, kann ein Teil des beladenen Kühlmediums aus dem Umlauf abgezogen werden und die Umlaufmenge des Kühlmediums durch Zugabe von unbeladenem Kühlmittel konstant gehalten werden.

Das Kühlmittel, welches als Kühlmedium zugegeben wird, besteht somit ebenfalls bevorzugt aus dem als Quench-Kühlmittel verwendeten aromatischen Kohlenwasserstoff-Lösungsmittel.

Der Kondensatstrom kann in den Kreislaufstrom des Quenches zurückgeführt werden. Dadurch können die im Kondensatstrom absorbierten $C_4$-Komponenten wieder in den Gasstrom gebracht und damit die Ausbeute erhöht werden. Geeignete Verdichter sind beispielsweise Turbo-, Drehkolben- und Hubkolbenverdichter. Die Verdichter können beispielsweise mit einem Elektromotor, einem Expander oder einer Gas- oder Dampfturbine angetrieben werden. Typische Verdichtungsverhältnisse (Austrittsdruck : Eintrittsdruck) pro Verdichterstufe liegen je nach Bauart zwischen 1,5 und 3,0. Die Abkühlung des verdichteten Gases erfolgt mit organischem Lösungsmittel gespülten Wärmetauschern oder organischen Quenchstufen, die beispielsweise als Rohrbündel-, Spiral oder Plattenwärmetauscher ausgeführt sein können. Als Kühlmittel kommen in den Wärmetauschern dabei Kühlwasser oder Wärmeträgeröle zum Einsatz. Daneben wird bevorzugt Luftkühlung unter Einsatz von Gebläsen eingesetzt.

Der Butadien, n-Butene, Sauerstoff, leicht siedende Kohlenwasserstoffe (Methan, Ethan, Ethen, Propan, Propen, n-Butan, iso-Butan), gegebenenfalls Wasserdampf, gegebenenfalls Kohlenstoffoxide sowie gegebenenfalls Inertgase und gegebenenfalls Spuren von Nebenkomponenten enthaltende Gasstrom c2 wird als Ausgangsstrom der weiteren Aufbereitung zugeführt.

In einem Schritt Da) werden nicht kondensierbare und leicht siedende Gasbestandteile, umfassend Sauerstoff, leicht siedenden Kohlenwasserstoffe (Methan, Ethan, Ethen, Propan, Propen), Kohlenstoffoxide und Inertgase in einer Absorptionskolonne aus dem Prozessgasstrom c2 durch Absorption der $C_4$-Kohlenwasserstoffe in einem aromatischen Kohlenwasserstofflösungsmittel als hochsiedendem Absorptionsmittel und nachfolgender Desorption der $C_4$-Kohlenwasserstoffe abgetrennt. Vorzugsweise umfasst der Schritt Da) die Schritte Daa) bis Dac):

Daa) Absorption der $C_4$-Kohlenwasserstoffe umfassend Butadien und n-Butene in einem aromatischen Kohlenwasserstofflösungsmittel als Absorptionsmittel, wobei ein mit $C_4$-Kohlenwasserstoffen beladener Absorptionsmittelstrom und der Gasstrom d2 erhalten werden,

Dab) Entfernung von Sauerstoff aus dem mit $C_4$-Kohlenwasserstoffen beladenen Absorptionsmittelstrom aus Schritt Daa) durch Strippung mit einem nicht kondensierbaren Gasstrom, und

Dac) Desorption der $C_4$-Kohlenwasserstoffe aus dem beladenen Absorptionsmittelstrom, wobei ein $C_4$-Produktgasstrom d1 erhalten wird, der im Wesentlichen aus $C_4$-Kohlenwasserstoffen besteht.

Dazu wird in der Absorptionsstufe der Gasstrom c2 mit dem Absorptionsmittel in Kontakt gebracht und werden die $C_4$-Kohlenwasserstoffe in dem Absorptionsmittel absorbiert, wobei ein mit $C_4$-Kohlenwasserstoffen beladenes Absorptionsmittel und ein die übrigen Gasbestandteile enthaltender Gasstrom d2 erhalten wird, der zumindest teilweise als Kreisgasstrom in die oxidative Dehydrierung zurückgeführt wird. In einer Desorptionsstufe werden die $C_4$-Kohlenwasserstoffe aus dem Absorptionsmittel wieder freigesetzt.

Als Absorptionsmittel werden organische Lösungsmittel, bevorzugt aromatische Kohlenwasserstoffe, besonders bevorzugt Toluol, o-Xylol, m-Xylol, p-Xylol, Mesitylen, alle möglichen Konstitutionsisomere von Mono-, Di- und Triethylbenzol sowie alle möglichen Konstitutionsisomere von Mono-, Di- und Triisopropylbenzol oder Gemische daraus, verwendet. Bevorzugt sind weiterhin aromatische Kohlenwasserstoffe mit einem Siedepunkt bei 1013,25 hPa von über 120 °C oder Gemische daraus. Insbesondere wird in der Abtrennstufe Da) das gleiche aromatische Kohlenwasserstofflösungsmittel eingesetzt wie in der vorhergehenden Abkühlstufe Ca), wenn in der Abkühlstufe Ca) ein organisches Lösungsmittel verwendet wird. Bevorzugte Absorptionsmittel sind Lösungsmittel, die ein Lösungsvermögen für organische Peroxide von mindestens 1000 ppm (mg aktiver Sauerstoff / kg Lösungsmittel) aufweisen. In einer bevorzugten Ausführungsform wird als Absorptionsmittel für die Absorption Mesitylen eingesetzt.

Die Absorptionsstufe kann in jeder beliebigen, dem Fachmann bekannten geeigneten Absorptionskolonne durchgeführt werden. Die Absorption kann durch einfaches Durchleiten des Produktgasstroms durch das Absorptionsmittel erfolgen. Sie kann aber auch in Kolonnen oder in Rotationsabsorbern erfolgen. Dabei kann im Gleichstrom, Gegenstrom oder Kreuzstrom gearbeitet werden. Bevorzugt wird die Absorption im Gegenstrom durchgeführt. Geeignete Absorptionskolonnen sind z.B. Bodenkolonnen mit Glocken-, Zentrifugal- und/oder Siebboden, Kolonnen mit strukturierten Packungen, z.B. Blechpackungen mit einer spezifischen Oberfläche von 100 bis 1000 $m^2/m^3$ wie Mellapak® 250 Y, und Füllkörperkolonnen. Es kommen aber auch Riesel- und Sprühtürme, Graphitblockabsorber, Oberflächenabsorber wie Dickschicht und Dünnschichtabsorber sowie Rotationskolonnen, Tellerwäscher, Kreuzschleierwäscher und Rotationswäscher in Betracht.

In einer Ausführungsform wird einer Absorptionskolonne im unteren Bereich der Butadien, n-Butene und die leichtsiedenden und nicht kondensierbaren Gasbestandteile enthaltende Gasstrom c2 zugeführt. Im oberen Bereich der Absorptionskolonne wird das Absorptionsmittel aufgegeben.

Am Kopf der Absorptionskolonne wird ein Gasstrom d2 abgezogen, der im Wesentlichen Sauerstoff, leicht siedende Kohlenwasserstoffe (Methan, Ethan, Ethen, Propan, Propen), das aromatische Kohlenwasserstofflösungsmit-tel, gegebenenfalls $C_4$-Kohlenwasserstoffe (Butan, Butene, Butadien), gegebenenfalls Inertgase, gegebenenfalls Kohlenstoffoxide und gegebenenfalls noch Wasserdampf enthält. Dieser Stoffstrom wird zumindest teilweise als Kreisgasstrom a2 dem ODH-Reaktor zugeführt. Damit lässt sich zum Beispiel der Eintrittsstrom des ODH-Reaktors auf den gewünschten $C_4$-Kohlenwasserstoffgehalt einstellen. Im Allgemeinen werden, gegebenenfalls nach Abtrennung eines Purgegasstroms, mindestens 30 Vol.-%, bevorzugt mindestens 50 Vol.-% des Gasstroms d2 als Kreisgasstrom a2 in die oxidative Dehydrierzone zurückgeführt.

Im Allgemeinen beträgt der Rückführstrom 10 bis 70 Vol.-%, bevorzugt 30 bis 60 Vol.-%, bezogen auf die Summe aller in die oxidative Dehydrierung B) eingespeisten Stoffströme.

Der Purgegasstrom kann einer thermischen oder katalytischen Nachverbrennung unterzogen werden. Insbesondere kann er in einem Kraftwerk thermisch verwertet werden.

Am Sumpf der Absorptionskolonne können in einer weiteren Kolonne durch die Spülung mit einem Gas Reste von im Absorptionsmittel gelöstem Sauerstoff ausgetragen werden. Der verbleibende Sauerstoffanteil ist vorzugsweise so klein, dass der die Desorptionskolonne verlassende und Butan, Buten sowie Butadien enthaltende Strom d1 nur noch maximal 100 ppm Sauerstoff enthält.

Das Ausstrippen des Sauerstoffs in Schritt Dab) kann in jeder beliebigen, dem Fachmann bekannten geeigneten Kolonne durchgeführt werden. Das Strippen kann durch einfaches Durchleiten von nicht kondensierbaren Gasen, vorzugsweise Inertgasen wie Stickstoff, durch die beladene Absorptionslösung erfolgen. Mit ausgestripptes C4 wird im oberen Teil der Absorptionskolonne zurück in die Absorptionslösung gewaschen, indem der Gasstrom in diese Absorptionskolonne zurück geleitet wird. Das kann sowohl durch eine Verrohrung der Stripperkolonne als auch eine direkte Montage der Stripperkolonne unterhalb der Absorberkolonne erfolgen. Da der Druck im Strippkolonnenteil und Absorptionskolonnenteil erfindungsgemäß gleich ist, kann diese direkte Kopplung erfolgen. Geeignete Stippkolonnen sind z.B. Bodenkolonnen mit Glocken-, Zentrifugal- und/oder Siebboden, Kolonnen mit strukturierten Packungen, z.B. Blechpackungen mit einer spezifischen Oberfläche von 100 bis 1000 $m^2/m^3$ wie Mellapak® 250 Y, und Füllkörperkolonnen. Es kommen aber auch Riesel- und Sprühtürme sowie Rotationskolonnen, Tellerwäscher, Kreuzschleierwäscher und Rotationswäscher in Betracht. Geeignete Gase sind zum Beispiel Stickstoff oder Methan.

Der mit $C_4$-Kohlenwasserstoffen beladene Absorptionsmittelstrom kann Wasser beinhalten. Dieses kann in einem Dekanter als Strom vom Absorptionsmittel abgetrennt, so dass ein Strom erhalten wird, der nur noch das im Absorptionsmittel eingelöste Wasser enthält.

Der mit $C_4$-Kohlenwasserstoffen beladene, weitestgehend vom Wasser befreite Absorptionsmittelstrom d2 kann in einem Wärmetauscher erwärmt werden und anschließend in eine Desorptionskolonne geleitet werden. In einer Verfahrensvariante wird der Desorptionsschritt Dc) durch Entspannung und/oder Erhitzen des beladenen Absorptionsmittels durchgeführt. Bevorzugte Verfahrensvariante ist die Nutzung eines Reboilers im Sumpf der Desorptionskolonne.

Das in der Desorptionsstufe regenerierte Absorptionsmittel kann in einem Wärmetauscher abgekühlt werden und in die Absorptionsstufe zurückgeführt werden. Im Prozessgasstrom befindliche Leichtsieder wie beispielsweise Ethan oder Propan sowie schwersiedende Komponenten wie Benzaldehyd, Maleinsäureanhydrid und Phthalsäureanhydrid, können sich im Kreislaufstrom anreichern. Um die Anreicherung zu begrenzen, kann ein Purgestrom abgezogen werden. Dieser kann in einer Destillationskolonne nach dem Stand der Technik in Leichtsieder, regeneriertes Absorbens und Schwersieder aufgetrennt werden.

Der im Wesentlichen aus n-Butan, n-Butenen und Butadien bestehende $C_4$-Produktgasstrom d1 enthält im Allgemeinen 20 bis 80 Vol.-% Butadien, 0 bis 80 Vol.-% n-Butan, 0 bis 10 Vol.-% 1-Buten, und 0 bis 50 Vol.-% 2-Butene, wobei die Gesamtmenge 100 Vol.-% ergibt. Weiterhin können geringe Mengen an iso-Butan enthalten sein.

Ein Teil des kondensierten, hauptsächlich $C_4$-Kohlenwasserstoffe enthaltenen Kopfaustrags der Desorptionskolonne wird in den Kolonnenkopf zurückgeführt, um die Trennleistung der Kolonne zu erhöhen.

Die den Kondensator verlassenden, flüssigen bzw. gasförmigen $C_4$-Produktströme können anschließend durch Extraktivdestillation im Schritt E) mit einem für Butadien selektiven Lösungsmittel in einen Butadien und das selektive Lösungsmittel enthaltenden Stoffstrom und einen n-Butene enthaltenden Stoffstrom aufgetrennt werden.

Die Extraktivdestillation kann beispielsweise, wie in "Erdöl und Kohle - Erdgas - Petrochemie", Band 34 (8), Seiten 343 bis 346 oder "Ullmanns Enzyklopädie der Technischen Chemie", Band 9, 4. Auflage 1975, Seiten 1 bis 18 beschrieben, durchgeführt werden. Hierzu wird der $C_4$-Produktgasstrom mit einem Extraktionsmittel, vorzugsweise einem N-Methylpyrrolidon (NMP)/Wasser-Gemisch, in einer Extraktionszone in Kontakt gebracht. Die Extraktionszone ist im Allgemeinen in Form einer Waschkolonne ausgeführt, welche Böden, Füllkörper oder Packungen als Einbauten enthält. Diese weist im Allgemeinen 30 bis 70 theoretische Trennstufen auf, damit eine hinreichend gute Trennwirkung erzielt wird. Vorzugsweise weist die Waschkolonne im Kolonnenkopf eine Rückwaschzone auf. Diese Rückwaschzone dient zur Rückgewinnung des in der Gasphase enthaltenen Extraktionsmittels mit Hilfe eines flüssigen Kohlenwasserstoffrücklaufs, wozu die Kopffraktion zuvor kondensiert wird. Das Massenverhältnis Extraktionsmittel zu $C_4$-Produktgasstrom im Zulauf der Extraktionszone beträgt im Allgemeinen 10 : 1 bis 20 : 1. Die Extraktivdestillation wird vorzugsweise bei einer Sumpftemperatur im Bereich von 100 bis 250 °C, insbesondere bei einer Temperatur im Bereich von 110 bis 210 °C, einer Kopftemperatur im Bereich von 10 bis 100·°C, insbesondere im Bereich von 20 bis 70·°C und einem Druck im Bereich von 1 bis 15 bar, insbesondere im Bereich von 3 bis 8 bar betrieben. Die Extraktivdestillationskolonne weist vorzugsweise 5 bis 70 theoretische Trennstufen auf.

Geeignete Extraktionsmittel sind Butyrolacton, Nitrile wie Acetonitril, Propionitril, Methoxypropionitril, Ketone wie Aceton, Furfural, N-alkylsubstituierte niedere aliphatische Säureamide wie Dimethylformamid, Diethylformamid, Dimethylacetamid, Diethylacetamid, N-Formylmorpholin, N-alkylsubstituierte zyklische Säureamide (Lactame) wie N-Alkylpyrrolidone, insbesondere N-Methylpyrrolidon (NMP). Im Allgemeinen werden alkylsubstituierte niedere aliphatische Säureamide oder N-alkylsubstituierte zyklische Säureamide verwendet. Besonders vorteilhaft sind Dimethylformamid, Acetonitril, Furfural und insbesondere NMP.

Es können jedoch auch Mischungen dieser Extraktionsmittel untereinander, z.B. von NMP und Acetonitril, Mischungen dieser Extraktionsmittel mit Co-Lösungsmitteln und/oder tert.-Butylether, z.B. Methyl-tert.-butylether, Ethyl-tert.-butylether, Propyl-tert.-butylether, n- oder iso-Butyl-tert.-butylether eingesetzt werden. Besonders geeignet ist NMP, bevorzugt in wässriger Lösung, vorzugsweise mit 0 bis 20 Gew.-% Wasser, besonders bevorzugt mit 7 bis 10 Gew.-% Wasser, insbesondere mit 8,3 Gew.-% Wasser.

Der Kopfproduktstrom der Extraktivdestillationskolonne enthält im Wesentlichen Butan und Butene und in geringen Mengen Butadien und wird gasförmig oder flüssig abgezogen. Im Allgemeinen enthält der im Wesentlichen aus n-Butan und 2-Buten bestehende Strom bis 100 Vol.-% n-Butan, 0 bis 50 Vol.-% 2-Buten und 0 bis 3 Vol.-% weitere Bestandteile wie Isobutan, Isobuten, Propan, Propen und $C_5^+$-Kohlenwasserstoffe.

Der im Wesentlichen aus n-Butan und 2-Butenen bestehende Strom kann ganz oder teilweise dem $C_4$-Feed des ODH-Reaktors zugeführt werden. Da die Buten-Isomere dieses Rückführstroms im Wesentlichen aus 2-Butenen bestehen, und 2-Butene im Allgemeinen langsamer zu Butadien oxidativ dehydriert werden als 1-Buten, kann dieser Rückführstrom vor der Zuführung in den ODH-Reaktor katalytisch isomerisiert werden. Dadurch kann die Isomerenverteilung entsprechend der im thermodynamischen Gleichgewicht vorliegenden Isomerenverteilung eingestellt werden.

In einem Schritt F) wird der Butadien und das selektive Lösungsmittel enthaltende Stoffstrom in einen im Wesentlichen aus dem selektiven Lösungsmittel bestehenden Stoffstrom und einen Butadien enthaltenden Stoffstrom destillativ aufgetrennt.

Der am Sumpf der Extraktivdestillationskolonne gewonnene Stoffstrom enthält im Allgemeinen das Extraktionsmittel, Wasser, Butadien und in geringen Anteilen Butene und Butan und wird einer Destillationskolonne zugeführt. In dieser kann über Kopf oder als Seitenabzug Butadien gewonnen werden. Am Sumpf der Destillationskolonne fällt ein Extraktionsmittel und gegebenenfalls Wasser enthaltender Stoffstrom an, wobei die Zusammensetzung des Extraktionsmittel und Wasser enthaltenden Stoffstroms der Zusammensetzung entspricht, wie sie der Extraktion zugegeben wird. Der Extraktionsmittel und Wasser enthaltende Stoffstrom wird bevorzugt in die Extraktivdestillation zurückgeleitet.

Falls das Butadien über einen Seitenabzug gewonnen wird, wird die so abgezogene Extraktionslösung wird in eine Desorptionszone überführt, wobei aus der Extraktionslösung das Butadien nochmals desorbiert und rückgewaschen wird. Die Desorptionszone kann beispielsweise in Form einer Waschkolonne ausgeführt sein, die 2 bis 30, bevorzugt 5 bis 20 theoretische Stufen und gegebenenfalls eine Rückwaschzone mit beispielsweise 4 theoretischen Stufen aufweist. Diese Rückwaschzone dient zur Rückgewinnung des in der Gasphase enthaltenen Extraktionsmittels mit Hilfe eines flüssigen Kohlenwasserstoffrücklaufs, wozu die Kopffraktion zuvor kondensiert wird. Als Einbauten sind Packungen, Böden oder Füllkörper vorgesehen. Die Destillation wird vorzugsweise bei einer Sumpftemperatur im Bereich von 100 bis 300 °C, insbesondere im Bereich von 150 bis 200 °C und einer Kopftemperatur im Bereich von 0 bis 70 °C, insbesondere im Bereich von 10 bis 50 °C durchgeführt. Der Druck in der Destillationskolonne liegt dabei vorzugsweise im Bereich von 1 bis 10 bar. Im Allgemeinen herrschen in der Desorptionszone gegenüber der Extraktionszone ein verminderter Druck und/oder eine erhöhte Temperatur.

Der am Kolonnenkopf gewonnene Wertproduktstrom enthält im Allgemeinen 90 bis 100 Vol.-% Butadien, 0 bis 10 Vol.-% 2-Buten und 0 bis 10 Vol.-% n-Butan und iso-Butan. Zur weiteren Aufreinigung des Butadiens kann eine weitere Destillation nach dem Stand der Technik durchgeführt werden.

**Beispiele**

Katalysatorherstellung

Es wurden 2 Lösungen A und B hergestellt.

Lösung A:

In einem 10 l-Edelstahltopf wurden 3200 g Wasser vorgelegt und unter Rühren mittels eines Ankerrührers 5,2 g einer KOH-Lösung (32 Gew.-% KOH) zugegeben. Die Lösung wurde auf 60 °C erwärmt. Nun wurden 1066 g einer Ammoniumheptamolybdatlösung (($NH_4$)$_6$Mo$_7$O2$_4$*4 H$_2$0, 54 Gew.-% Mo) portionsweise über einen Zeitraum von 10 Minuten zugegeben. Die erhaltene Suspension wurde noch 10 Minuten nachgerührt.

Lösung B:

In einem 5 l-Edelstahltopf wurden 1771 g einer Kobalt(II)nitratlösung (12,3 Gew.-% Co) vorgelegt und unter Rühren (Ankerrührer) auf 60 °C erhitzt. Nun wurden 645 g einer Eisen(III)nitratlösung (13,7 Gew.-% Fe) über einen Zeitraum von 10 Minuten portionsweise unter Aufrechterhaltung der Temperatur zugegeben. Die entstandene Lösung wurde 10 min nachgerührt. Nun wurden 619 g einer Bismutnitratlösung (10,7 Gew.-% Bi) unter Aufrechterhaltung der Temperatur zugegeben. Nach weiteren 10 Minuten Nachrühren wurden 109 g Chrom(III)nitrat portionsweise fest zugegeben und die entstandene dunkelrote Lösung 10 min weitergerührt.

Unter Beibehaltung von 60 °C wurde innerhalb von 15 min die Lösung B zur Lösung A mittels Schlauchpumpe zugegeben. Während der Zugabe und danach wurde mittels eines Intensivmischers (Ultra-Turrax) gerührt. Nach vollendeter Zugabe wurde noch 5 Minuten weitergerührt.

Die erhaltene Suspension wurde in einem Sprühturm der Fa. NIRO (Sprühkopf-Nr. FOA1, Drehzahl 25000 U/min) über einen Zeitraum von 1,5 h sprühgetrocknet. Dabei wurde die Vorlagetemperatur bei 60 °C gehalten. Die Gaseingangstemperatur des Sprühturmes betrug 300 °C, die Gasausgangstemperatur 110 °C. Das erhaltene Pulver hatte eine Partikelgröße (d50) kleiner 40 µm.

Das erhaltene Pulver wurde mit 1 Gew.-% Graphit vermischt, zweimal mit 9 bar Pressdruck kompaktiert und durch ein Sieb mit Maschenweite 0,8 mm zerkleinert. Der Split wurde wiederum mit 2 Gew.-% Graphit vermengt und die Mischung mit einer Kilian S100 Tablettenpresse in Ringe 5 x 3 x 2 mm (Außendurchmesser x Länge x Innendurchmesser) gepresst.

Der erhaltene Katalysatorvorläufer wurde chargenweise (500 g) in einem Umluftofen der Firma Heraeus, DE (Typ K, 750/2 S, Innenvolumen 55 l) kalziniert. Folgendes Programm wurde dafür verwendet:

- Aufheizen in 72 Minuten auf 130 °C, 72 Minuten halten
- Aufheizen in 36 Minuten auf 190 °C, 72 Minuten halten
- Aufheizen in 36 Minuten auf 220 °C, 72 Minuten halten
- Aufheizen in 36 Minuten auf 265 °C, 72 Minuten halten
- Aufheizen in 93 Minuten auf 380 °C, 187 Minuten halten
- Aufheizen in 93 Minuten auf 430 °C, 187 Minuten halten
- Aufheizen in 93 Minuten auf 490 °C, 467 Minuten halten

Nach der Kalzination wurden der Katalysator der berechneten Stöchiometrie Mo$_{12}$Co$_7$Fe$_3$Bi0.$_6$K0.0$_8$Cr0.$_5$O$_x$ erhalten.

Die kalzinierten Ringe wurden zu einem Pulver vermahlen. Mit dieser Vorläufermasse wurden in drei Chargen Trägerkörper (Steatitringe mit Abmessungen 5 x 3 x 2 mm (Außendurchmesser x Höhe x Innendurchmesser) beschichtet. Dazu wurden jeweils 1054 g des Trägers in einer Dragiertrommel (25,5 cm Durchmesser, Neigungswinkel der Trommelmittelachse gegen die Horizontale = 30°) vorgelegt. Die Trommel wurde in Rotation versetzt (36 U/min). Über eine mit Druckluft betriebenen Zerstäuberdüse wurden über ca. 25 Minuten hinweg ca. 60 ml flüssiges Bindemittel (Mischung Glycerin : Wasser 1:3) auf den Träger gesprüht (Sprühluft 200 Nl/h). Die Düse war dabei derart installiert, dass der Sprühkegel die in der Trommel beförderten Trägerkörper in der oberen Hälfte der Abrollstrecke benetzte. Insgesamt 191 g der feinpulvrigen Vorläufermasse des gemahlenen Katalysators wurden über eine Pulverschnecke in die Trommel eingetragen, wobei der Punkt der Pulverzugabe innerhalb der Abrollstrecke, aber unterhalb des Sprühkegels lag. Die Pulverzugabe wurde dabei so dosiert, dass eine gleichmäßige Verteilung des Pulvers auf der Oberfläche entstand. Nach Abschluss der Beschichtung wurde der entstandene Schalenkatalysator aus Vorläufermasse und dem Trägerkörper in einem Trockenschrank bei 300 °C für 4 Stunden getrocknet. Der Aktivmassengehalt betrug 15 Gew.-%.

Dehydrierversuche

In einem Screening-Reaktor wurden Dehydrierungsversuche durchgeführt. Der Screening-Reaktor war ein Salzbadreaktor mit einer Länge von 125 cm und einem Innendurchmesser von 14,9 mm und einer Thermohülse mit einem Außendurchmesser von 3,17 mm. In der Thermohülse befand sich ein Mehrfachthermoelement mit 7 Messstellen. Die untersten 4 Messstellen hatten einen Abstand von 10 cm und die obersten 4 Messstellen einen Abstand von 5 cm. Das Butane/Butene-Gemisch wurde bei circa 10 bar flüssig durch einen Koriolis-Flussmesser dosiert, in einem statischen Mischer vermischt und anschließend in einer beheizten Verdampferstrecke entspannt und verdampft. Dieses Gas wurde nun mit Stickstoff gemischt und in einem Vorheizer mit einer Steatitschüttung geleitet. Wasser wurde flüssig dosiert und in einer Verdampferwendel in einem Luftstrom verdampft. Das Luft/Wasserdampfgemisch wurde im unteren Bereich des Vorheizers mit dem $N_2$/Raffinat-II/Butan-Gemisch vereinigt. Das komplett vermischte Eduktgas wurde dann dem Reaktor zugeführt, wobei ein Analysenstrom für die online-GC-Messung abgezogen werden kann. Aus dem Produktgas, welches den Reaktor verlässt, wird ebenfalls ein Analysenstrom abgezogen, welcher per online-GC-Messung analysiert werden kann. Ein Druckregelventil schließt sich hinter dem Abzweig der Analysenleitung an, welches das Druckniveau vor dem Reaktor auf 1 bar Überdruck einstellt.

Auf den Katalysatorstuhl am unteren Ende des Screening-Reaktors wurde eine 9,5 cm lange Nachschüttung bestehend aus 24 g Steatitkugeln mit einem Durchmesser von 3,5-4,5 mm gefüllt. Danach wurden 120 g des Schalenkatalysators in den Reaktor gefüllt (108 ml Schüttvolumen, 65 cm Schütthöhe). An die Katalysatorschüttung schloss sich eine 6 cm lange Vorschüttung bestehend aus 16 g Steatitkugeln mit einem Durchmesser von 3,5-4,5 mm an.

Tabelle 1: Zusammensetzung des C4 haltigen Gases

| i-Butan | n-Butan | t-2-Buten | c-2-Buten | 1-Buten | i-Buten |
|---------|---------|-----------|-----------|---------|---------|
| 4,0 mol.-% | 20,5 mol.-% | 39,5 mol.-% | 16,0 mol.-% | 16,5 mol.-% | 3,5 mol.-% |

Der Katalysator wurde über 24 h mit einem Gemisch von 10 Vol.-% Sauerstoff, 80 Vol.-% Stickstoff und 10 Vol.-% Wasserdampf (Gesamtvolumenstrom 150 Nl/h) bei 400 °C aktiviert. Danach wurde der Reaktor mit 150 NL/h eines Reaktionsgases (10,4 Vol.-% C4-haltiges Gas, Zusammensetzung siehe Tabelle 1; 12,2 Vol.-% $O_2$; 10,3 Vol.-% Dampf; Rest $N_2$) bei einer Salzbadtemperatur von 380 °C für 6 Tage eingelaufen. In dieser Zeit wurden mittels online-GC die Bestandteile des Edukt- und Produktgases untersucht. Die in den Beispielen berechneten Größen Umsatz (X) und Selektivität (S) wurden wie folgt bestimmt:

$$X = \frac{mol(Butene_{ein}) - mol(Butene_{aus})}{mol(Butene_{ein})}$$

$$S = \frac{mol(Butadien_{aus}) - mol(Butadien_{ein})}{mol(Butene_{ein}) - mol(Butene_{aus})}$$

wobei mol(XXXein) die Stoffmenge der Komponente XXX am Reaktoreingang ist, mol(XXXaus) die Stoffmenge der Komponente XXX am Reaktorausgang ist und Butene die Summe aus 1-Buten, cis-2-Buten, trans-2-Buten und iso-Buten darstellt.

Beispiel 1

Für 24 Stunden wurde der Reaktor mit den in Tabelle 2 angegebenen Eduktgas-Zusammensetzung und den oben genannten Reaktionsbedingungen betrieben. In diesem Zeitraum ergab sich bei einem Butene-Umsatz von 87,2 % sich eine Selektivität zu Butadien von 84%.

Referenzbeispiel 2

Im Anschluss wurde für weitere 24 Stunden der Reaktor mit den in Tabelle 2 angegebenen Feed-Zusammensetzung und den oben genannten Reaktionsbedingungen betrieben. Zusätzlich wurden in dieser Zeit 0,34 Vol.-% Mesitylen dem Eduktgas zugegeben. In diesem Zeitraum fiel der Butene-Umsatz auf 63,4 % bei einer Selektivität zu Butadien von 87,1 %.

Referenzbeispiel 3

Im Anschluss wurde für weitere 24 Stunden der Reaktor mit den in Tabelle 2 angegebenen Feed-Zusammensetzung und den oben genannten Reaktionsbedingungen betrieben. Zusätzlich wurden in dieser Zeit 0,43 Vol.-% Mesitylen dem Eduktgas zugegeben. In diesem Zeitraum fiel der Butene-Umsatz auf 57,1 % bei einer Selektivität zu Butadien von 85,8 %.

Referenzbeispiel 4

Im Anschluss wurde für weitere 24 Stunden der Reaktor mit den in Tabelle 2 angegebenen Feed-Zusammensetzung und den oben genannten Reaktionsbedingungen betrieben. Zusätzlich wurden in dieser Zeit 0,21 vol.-% Mesitylen dem Eduktgas zugegeben. In diesem Zeitraum stieg der Butene-Umsatz auf 71,4 % bei einer Selektivität zu Butadien von 86,9 %.

Beispiel 5

Im Anschluss wurde für weitere 24 Stunden der Reaktor mit der in Tabelle 2 angegebenen Feed-Zusammensetzung unter den oben genannten Reaktionsbedingungen betrieben. Es wurde in dieser Zeit kein Mesitylen dem Eduktgas zugegeben. In diesem Zeitraum stieg der Butene-Umsatz auf 85,6 % bei einer Selektivität zu Butadien von 87,6 %.

Tabelle 2: Eduktgas-Zusammensetzung, Butene-Umsatz und Butadienselektivität

| Beispiel | C4-Gas [vol.-%] | O2 [vol.-%] | N2 [vol.-%] | H2O [vol.-%] | Mesitylen [vol.-%] | Umsatz der Butene [%] | Selektivität zu Butadien [%] |
|----------|-----------------|-------------|-------------|--------------|--------------------|-----------------------|------------------------------|
| 1 | 10,4 | 11,9 | rest | 10,2 | - | 87,2 | 84,0 |
| 2 | 10,2 | 11,9 | rest | 10,3 | 0,34 | 63,4 | 87,1 |
| 3 | 10,6 | 12,0 | rest | 10,2 | 0,43 | 57,1 | 85,8 |
| 4 | 10,3 | 12,0 | rest | 10,1 | 0,21 | 71,4 | 86,9 |
| 5 | 10,1 | 12,3 | rest | 10,4 | - | 85,6 | 87,6 |

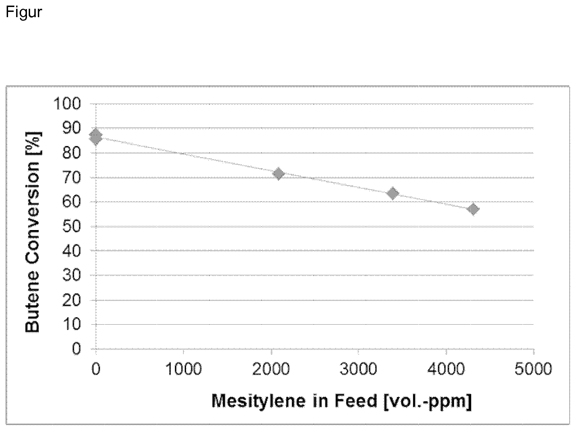

**Patentansprüche**

1.  Verfahren zur Herstellung von Butadien aus n-Butenen mit den Schritten:

    A) Bereitstellung eines n-Butene enthaltenden Einsatzgasstroms a1,
    B) Einspeisung des n-Butene enthaltenden Einsatzgasstromes a1, eines sauerstoffhaltigen Gases und eines sauerstoffhaltigen Kreisgasstroms a2 in mindestens eine oxidative Dehydrierzone und oxidative Dehydrierung von n-Butenen zu Butadien, wobei ein Produktgasstrom b enthaltend Butadien, nicht umgesetzte n-Butene, Wasserdampf, Sauerstoff, leicht siedende Kohlenwasserstoffe, hochsiedende Nebenkomponenten, gegebenenfalls Kohlenstoffoxide und gegebenenfalls Inertgase erhalten wird,
    Ca) Abkühlung des Produktgasstroms b und gegebenenfalls zumindest teilweise Abtrennung von hochsiedenden Nebenkomponenten und von Wasserdampf, wobei ein Produktgasstrom b' erhalten wird,
    Cb) Kompression und Kühlung des Produktgasstroms b' in mindestens einer Kompressions- und Kühlungsstufe, wobei mindestens ein wässriger Kondensatstrom c1 und ein Gasstrom c2 enthaltend Butadien, n-Butene, Wasserdampf, Sauerstoff, leicht siedende Kohlenwasserstoffe, gegebenenfalls Kohlenstoffoxide und gegebenenfalls Inertgase erhalten wird,
Da) Absorption der $C_4$-Kohlenwasserstoffe umfassend Butadien und n-Butene in einem aromatischen Kohlenwasserstofflösungsmittel als Absorptionsmittel und Abtrennung von nicht kondensierbaren und leicht siedenden Gasbestandteilen umfassend Sauerstoff, leicht siedende Kohlenwasserstoffe, gegebenenfalls Kohlenstoffoxide, aromatisches Kohlenwasserstofflösungsmittel und gegebenenfalls Inertgase als Gasstrom d2 aus dem Gasstrom c2 , wobei ein mit $C_4$-Kohlenwasserstoffen beladener Absorptionsmittelstrom und der Gasstrom d2 erhalten werden, und anschließende Desorption der $C_4$-Kohlenwasserstoffe aus dem beladenen Absorptionsmittelstrom, wobei ein $C_4$-Produktgasstrom d1 erhalten wird,
Db) zumindest teilweise Rückführung des Gasstroms d2 als Kreisgasstrom a2 in die oxidative Dehydrierzone,

**dadurch gekennzeichnet, dass** der Gehalt an aromatischem Kohlenwasserstofflösungsmittel im Kreisgasstrom a2 auf weniger als 0,2 Vol.-% begrenzt wird.

2. Verfahren nach Anspruch 1, **dadurch gekennzeichnet, dass** das in Schritt Da) als Absorptionsmittel eingesetzte aromatische Kohlenwasserstofflösungsmittel ausgewählt ist aus der Gruppe bestehend aus Toluol, o-, m-, p-Xylol, Mesitylen, Mono-, Di- und Triethylbenzol und Mono-, Di- und Triisopropylbenzol und deren Gemischen.

3. Verfahren nach Anspruch 2, **dadurch gekennzeichnet, dass** das aromatische Kohlenwasserstofflösungsmittel Mesitylen ist.

4. Verfahren nach einem der Ansprüche 1 bis 3, **dadurch gekennzeichnet, dass** der Gehalt an aromatischem Kohlenwasserstofflösungsmittel im Kreisgasstrom a2 auf weniger als 0,2 Vol.-% dadurch begrenzt wird, dass der der die Abtrennstufe Da) verlassende Gasstrom d2 in einer weiteren Kolonne mit einem flüssigen Absorbens für das aromatische Kohlenwasserstofflösungsmittel in Kontakt gebracht wird.

5. Verfahren nach einem der Ansprüche 1 bis 4, **dadurch gekennzeichnet, dass** der Gehalt an aromatischem Kohlenwasserstofflösungsmittel im Kreisgasstrom a2 auf weniger als 0,2 Vol.-% dadurch begrenzt wird, dass der der die Abtrennstufe Da) verlassende Gasstrom d2 mit einem festen Adsorbens in Kontakt gebracht wird, das das aromatische Kohlenwasserstofflösungsmittel adsorbiert.

6. Verfahren nach einem der Ansprüche 1 bis 5, **dadurch gekennzeichnet, dass** der Gehalt an aromatischem Kohlenwasserstofflösungsmittel im Kreisgasstrom a2 auf weniger als 0,2 Vol.-% dadurch begrenzt, dass der die Abtrennstufe Da) verlassende Gasstrom d2 mit einem als Kondensator ausgeführter Wärmetauscher in Kontakt gebracht wird, wobei durch Abkühlung das in dem Strom d2 enthaltene aromatische Kohlenwasserstofflösungsmittel zumindest teilweise als Flüssigphase abgeschieden wird.

7. Verfahren nach einem der Ansprüche 1 bis 6, **dadurch gekennzeichnet, dass** der Gehalt an aromatischem Kohlenwasserstofflösungsmittel im Kreisgasstroms a2 auf weniger als 0,2 Vol.-% dadurch begrenzt wird, dass eine thermische oder katalytische Nachverbrennung des aromatischen Kohlenwasserstofflösungsmittels durchgeführt wird.

8. Verfahren nach einem der Ansprüche 1 bis 7, **dadurch gekennzeichnet, dass** der Gehalt an aromatischem Kohlenwasserstofflösungsmittel im Kreisgasstroms a2 auf weniger als 0,2 Vol.-% dadurch begrenzt wird, dass in der in Schritt Da) eingesetzten Absorptionskolonne eine Vorrichtungen vorgesehen wird, die den Mitriss von flüssigen Bestandteilen aus der Absorptionskolonne in den Gasstrom d2 verringert.

9. Verfahren nach einem der Ansprüche 1 bis 6, **dadurch gekennzeichnet, dass** der Anteil des Kreisgasstroms a2 10 bis 70 Vol.-%, bezogen auf die Summe aller in die oxidative Dehydrierzone eingespeisten Gasströme, beträgt.

10. Verfahren nach einem der Ansprüche 1 bis 7, **gekennzeichnet durch** die zusätzlichen Schritte:

E) Auftrennung des $C_4$-Produktstroms d1 durch Extraktivdestillation mit einem für Butadien selektiven Lösungsmittel in einen Butadien und das selektive Lösungsmittel enthaltenden Stoffstrom e1 und einen n-Butene enthaltenden Stoffstrom e2;
F) Destillation des Butadien und das selektive Lösungsmittel enthaltenden Stoffstroms e2 in einen das selektive Lösungsmittel enthaltenden Stoffstrom f1 und einen Butadien enthaltenden Stoffstrom f2.

11. Verfahren nach einem der Ansprüche 1 bis 8, **dadurch gekennzeichnet, dass** der Schritt Da) die Schritte Daa) bis Dac) umfasst:

Daa) Absorption der $C_4$-Kohlenwasserstoffe umfassend Butadien und n-Butene in dem aromatischen Kohlenwasserstofflösungsmittel als Absorptionsmittel, wobei ein mit $C_4$-Kohlenwasserstoffen beladener Absorptionsmittelstrom und der Gasstrom d2 erhalten werden,
Dab) Entfernung von Sauerstoff aus dem mit $C_4$-Kohlenwasserstoffen beladenen Absorptionsmittelstrom aus Schritt Daa) durch Strippung mit einem nicht kondensierbaren Gasstrom, und
Dac) Desorption der $C_4$-Kohlenwasserstoffe aus dem beladenen Absorptionsmittelstrom, wobei ein $C_4$-Produktgasstrom d1 erhalten wird, der im Wesentlichen aus $C_4$-Kohlenwasserstoffen besteht.

**Claims**

1. A process for preparing butadiene from n-butenes, comprising the steps of:

A) providing an input gas stream a1 comprising n-butenes;
B) feeding the input gas stream a1 comprising n-butenes, an oxygenous gas and an oxygenous cycle gas stream a2 into at least one oxidative dehydrogenation zone and oxidatively dehydrogenating n-butenes to butadiene, giving a product gas stream b comprising butadiene, unconverted n-butenes, steam, oxygen, low-boiling hydrocarbons and high-boiling secondary components, with or without carbon oxides and with or without inert gases;
Ca) cooling the product gas stream b and optionally at least partly removing high-boiling secondary components and steam, giving a product gas stream b',
Cb) compressing and cooling the product gas stream b' in at least one compression and cooling stage, giving at least one aqueous condensate stream c1 and one gas stream c2 comprising butadiene, n-butenes, steam, oxygen and low-boiling hydrocarbons, with or without carbon oxides and with or without inert gases,
Da) absorbing the C4 hydrocarbons comprising butadiene and n-butenes in an aromatic hydrocarbon solvent as an absorbent and removing uncondensable and low-boiling gas constituents comprising oxygen, low-boiling hydrocarbons, any carbon oxides, aromatic hydrocarbon solvent and any inert gases as gas stream d2 from the gas stream c2, giving an absorbent stream laden with $C_4$ hydrocarbons and the gas stream d2, and then desorbing the $C_4$ hydrocarbons from the laden absorbent stream, giving a $C_4$ product gas stream d1,
Db) at least partly recycling the gas stream d2 as cycle gas stream a2 into the oxidative dehydrogenation zone,

wherein the content of aromatic hydrocarbon solvent in the cycle gas stream a2 is limited to less than 0.2% by volume.

2. The process according to claim 1, wherein the aromatic hydrocarbon solvent used as the absorbent in step Da) is selected from the group consisting of toluene, o-, m-, p-xylene, mesitylene, mono-, di- and triethylbenzene and mono-, di- and triisopropylbenzene, and mixtures thereof.

3. The process according to claim 2, wherein the aromatic hydrocarbon solvent is mesitylene.

4. The process according to any of claims 1 to 3, wherein the content of aromatic hydrocarbon solvent in the cycle gas stream a2 is limited to less than 0.2% by volume by contacting the gas stream d2 which leaves the removal stage Da) with a liquid absorbent for the aromatic hydrocarbon solvent in a further column.

5. The process according to any of claims 1 to 4, wherein the content of aromatic hydrocarbon solvent in the cycle gas stream a2 is limited to less than 0.2% by volume by contacting the gas stream d2 which leaves the removal stage Da) with a solid adsorbent which adsorbs the aromatic hydrocarbon solvent.

6. The process according to any of claims 1 to 5, wherein the content of aromatic hydrocarbon solvent in the cycle gas stream a2 is limited to less than 0.2% by volume by contacting the gas stream d2 which leaves the removal stage Da) with a heat exchanger in the form of a condenser, with at least partial deposition of the aromatic hydrocarbon solvent present in stream d2 as a liquid phase through cooling.

7. The process according to any of claims 1 to 6, wherein the content of aromatic hydrocarbon solvent in the cycle gas stream a2 is limited to less than 0.2% by volume by conducting a thermal or catalytic postcombustion of the aromatic hydrocarbon solvent.

8. The process according to any of claims 1 to 7, wherein the content of aromatic hydrocarbon solvent in the cycle gas stream a2 is limited to less than 0.2% by volume by providing, in the absorption column used in step Da), an apparatus which reduces the entrainment of liquid constituents from the absorption column into the gas stream d2.

9. The process according to any of claims 1 to 6, wherein the proportion of the cycle gas stream a2 is 10 to 70% by volume, based on the sum total of all the gas streams fed into the oxidative dehydrogenation zone.

10. The process according to any of claims 1 to 7, **characterized by** the additional steps of:

E) separating the $C_4$ product stream d1 by extractive distillation with a butadiene-selective solvent into a stream e1 comprising butadiene and the selective solvent and a stream e2 comprising n-butenes;
F) distilling the stream e2 comprising butadiene and the selective solvent to give a stream f1 comprising the selective solvent and a stream f2 comprising butadiene.

11. The process according to any of claims 1 to 8, wherein step Da) comprises steps Daa) to Dac):

Daa) absorbing the $C_4$ hydrocarbons comprising butadiene and n-butenes in the aromatic hydrocarbon solvent as an absorbent, giving an absorbent stream laden with $C_4$ hydrocarbons and the gas stream d2,
Dab) removing oxygen from the absorbent stream laden with $C_4$ hydrocarbons from step Daa) by stripping with an uncondensable gas stream, and
Dac) desorbing the $C_4$ hydrocarbons from the laden absorbent stream, giving a $C_4$ product gas stream d1 consisting essentially of $C_4$ hydrocarbons.

## Revendications

1. Procédé de fabrication de butadiène à partir de n-butènes comprenant les étapes suivantes :

A) la préparation d'un courant gazeux d'entrée contenant des n-butènes a1,
B) l'introduction du courant gazeux d'entrée contenant des n-butènes a1, d'un gaz contenant de l'oxygène et d'un courant gazeux circulaire contenant de l'oxygène a2 dans au moins une zone de déshydrogénation oxydative et la déshydrogénation oxydative de n-butènes en butadiène, un courant gazeux de produits b contenant du butadiène, des n-butènes non réagis, de la vapeur d'eau, de l'oxygène, des hydrocarbures de faible point d'ébullition, des composants secondaires de point d'ébullition élevé, éventuellement des oxydes de carbone et éventuellement des gaz inertes étant obtenu,
Ca) le refroidissement du courant gazeux de produits b et éventuellement la séparation au moins partielle de composants secondaires de point d'ébullition élevé et de vapeur d'eau, un courant gazeux de produits b' étant obtenu,
Cb) la compression et le refroidissement du courant gazeux de produits b' dans au moins une étape de compression et de refroidissement, au moins un courant de condensat aqueux c1 et un courant gazeux c2 contenant du butadiène, des n-butènes, de la vapeur d'eau, de l'oxygène, des hydrocarbures de faible point d'ébullition, éventuellement des oxydes de carbone et éventuellement des gaz inertes étant obtenus,
Da) l'absorption des hydrocarbures en $C_4$ comprenant du butadiène et des n-butènes dans un solvant hydrocarboné aromatique en tant qu'agent d'absorption et la séparation de constituants gazeux non condensables et de faible point d'ébullition comprenant de l'oxygène, des hydrocarbures de faible point d'ébullition, éventuellement des oxydes de carbone, le solvant hydrocarboné aromatique et éventuellement des gaz inertes en tant que courant gazeux d2 du courant gazeux c2, un courant d'agent d'absorption chargé avec des hydrocarbures en $C_4$ et le courant gazeux d2 étant obtenus, puis la désorption des hydrocarbures en $C_4$ du courant d'agent d'absorption chargé, un courant gazeux de produits en $C_4$ d1 étant obtenu,
Db) le recyclage au moins partiel du courant gazeux d2 en tant que courant de gaz circulaire a2 dans la zone de déshydrogénation oxydative,

caractérisé en ce la teneur en solvant hydrocarboné aromatique dans le courant gazeux circulaire a2 est limitée à moins de 0,2 % en volume.

2. Procédé selon la revendication 1, **caractérisé en ce que** le solvant hydrocarboné aromatique utilisé en tant qu'agent d'absorption à l'étape Da) est choisi dans le groupe constitué par le toluène, l'o-, le m-, le p-xylène, le mésitylène, le mono-, di- et triéthylbenzène et le mono-, di- et triisopropylbenzène et leurs mélanges.

**3.** Procédé selon la revendication 2, **caractérisé en ce que** le solvant hydrocarboné aromatique est le mésitylène.

**4.** Procédé selon l'une quelconque des revendications 1 à 3, **caractérisé en ce que** la teneur en solvant hydrocarboné aromatique dans le courant gazeux circulaire a2 est limitée à moins de 0,2 % en volume en mettant en contact le courant gazeux d2 quittant l'étape de séparation Da) dans une autre colonne avec un absorbant liquide pour le solvant hydrocarboné aromatique.

**5.** Procédé selon l'une quelconque des revendications 1 à 4, **caractérisé en ce que** la teneur en solvant hydrocarboné aromatique dans le courant gazeux circulaire a2 est limitée à moins de 0,2 % en volume en mettant en contact le courant gazeux d2 quittant l'étape de séparation Da) avec un adsorbant solide qui adsorbe le solvant hydrocarboné aromatique.

**6.** Procédé selon l'une quelconque des revendications 1 à 5, **caractérisé en ce que** la teneur en solvant hydrocarboné aromatique dans le courant gazeux circulaire a2 est limitée à moins de 0,2 % en volume en mettant en contact le courant gazeux d2 quittant l'étape de séparation Da) avec un échangeur de chaleur configuré sous la forme d'un condensateur, le solvant hydrocarboné aromatique contenu dans le courant d2 étant au moins partiellement séparé en tant que phase liquide par refroidissement.

**7.** Procédé selon l'une quelconque des revendications 1 à 6, **caractérisé en ce que** la teneur en solvant hydrocarboné aromatique dans le courant gazeux circulaire a2 est limitée à moins de 0,2 % en volume en réalisant une postcombustion thermique ou catalytique du solvant hydrocarboné aromatique.

**8.** Procédé selon l'une quelconque des revendications 1 à 7, **caractérisé en ce que** la teneur en solvant hydrocarboné aromatique dans le courant gazeux circulaire a2 est limitée à moins de 0,2 % en volume en prévoyant dans la colonne d'absorption utilisée à l'étape Da) un dispositif qui réduit l'entraînement de constituants liquides de la colonne d'absorption dans le courant gazeux d2.

**9.** Procédé selon l'une quelconque des revendications 1 à 6, **caractérisé en ce que** la proportion du courant gazeux circulaire a2 est de 10 à 70 % en volume, par rapport à la somme de tous les courants gazeux introduits dans la zone de déshydrogénation oxydative.

**10.** Procédé selon l'une quelconque des revendications 1 à 7, **caractérisé par** les étapes supplémentaires suivantes :

E) la séparation du courant de produits en $C_4$ d1 par distillation extractive avec un solvant sélectif pour le butadiène en un courant de matière e1 contenant le butadiène et le solvant sélectif et un courant de matière e2 contenant les n-butènes ;
F) la distillation du courant de matière e2 contenant le butadiène et le solvant sélectif en un courant de matière f1 contenant le solvant sélectif et un courant de matière f2 contenant le butadiène.

**11.** Procédé selon l'une quelconque des revendications 1 à 8, **caractérisé en ce que** l'étape Da) comprend les étapes Daa) à Dac) :

Daa) l'absorption des hydrocarbures en $C_4$ comprenant le butadiène et les n-butènes dans le solvant hydrocarboné aromatique en tant qu'agent d'absorption, un courant d'agent d'absorption chargé avec les hydrocarbures en $C_4$ et le courant gazeux d2 étant obtenus,
Dab) l'élimination de l'oxygène du courant d'agent d'absorption chargé avec les hydrocarbures en $C_4$ de l'étape Daa) par extraction avec un courant gazeux non condensable, et
Dac) la désorption des hydrocarbures en $C_4$ du courant d'agent d'absorption chargé, un courant gazeux de produits en $C_4$ d1 étant obtenu, qui est essentiellement constitué d'hydrocarbures en $C_4$.

Figur

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20120130137 A1
- US 20120130137 A
- JP 2011006381 A
- KR 20130036467
- KR 20130036468
- JP 2011001341 A
- JP 2010090083 A
- JP 2012072086 B
- US 3890123 A
- US 4141706 A

- US 4547615 A
- US 4424141 A
- DE 2530959 A
- US 3911039 A
- DE 2447825 A
- US 4423281 A
- US 4336409 A
- DE 2600128 A
- DE 2440329 A

**In der Beschreibung aufgeführte Nicht-Patentliteratur**

- **JUNG et al.** *Catal. Surv. Asia,* 2009, vol. 13, 78-93
- *Applied Catalysis A: General,* 2007, vol. 317, 244-249
- **D. S. ALEXANDER.** *Industrial and Engineering Chemistry,* 1959, vol. 51, 733-738
- Ullmann's Encyclopedia of Industrial Chemistry. 2011, vol. 7, 209-210
- ULLMANN'S ENCYCLOPEDIA OF INDUSTRIAL CHEMISTRY. vol. 3, 527-528
- **BEISPIEL PRASAD et al.** *Catal. Rev. Sci. Eng.,* 1984, vol. 26, 1

- *Trimm, Appl. Catal.,* 1983, vol. 7, 249
- **ARAI et al.** *Catal. Today,* 1995, vol. 26, 217
- **CENTI.** *J. Mol. Catal. A,* 2001, vol. 173, 287
- **KIRCHNEROVA.** *Kor. J. Chem. Eng.,* 1999, vol. 16, 427
- **CIUPARU et al.** *Catal. Rev. Sci. Eng.,* 2002, vol. 44, 593
- *Erdöl und Kohle - Erdgas - Petrochemie,* vol. 34 (8), 343-346
- Ullmanns Enzyklopädie der Technischen Chemie. 1975, vol. 9, 1-18